(19) Europäisches Patentamt
European Patent Office
Office européen des brevets

(11) EP 3 575 415 B1

(12) EUROPEAN PATENT SPECIFICATION

(45) Date of publication and mention of the grant of the patent:
**07.07.2021 Bulletin 2021/27**

(51) Int Cl.:
***C12Q 1/6806*** *(2018.01)* ***C12Q 1/6853*** *(2018.01)*
***C12N 15/10*** *(2006.01)*

(21) Application number: **19177151.8**

(22) Date of filing: **29.05.2019**

# (54) METHOD FOR SYNTHESIZING CDNA, METHOD FOR DETECTING TARGET RNA AND REAGENT KIT

VERFAHREN ZUR SYNTHESE VON CDNA, VERFAHREN ZUR ERKENNUNG VON ZIEL-RNA UND REAGENZKIT

PROCÉDÉ DE SYNTHÈSE D’ADNC, PROCÉDÉ DE DÉTECTION DE D’ARN CIBLE ET KIT DE RÉACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2018 JP 2018103590**

(43) Date of publication of application:
**04.12.2019 Bulletin 2019/49**

(73) Proprietor: **SYSMEX CORPORATION**
**Kobe-shi**
**Hyogo 651-0073 (JP)**

(72) Inventors:
- **Masumoto, Kanako**
  **Hyogo, 651-0073 (JP)**
- **Aihara, Yuki**
  **Hyogo, 651-0073 (JP)**

(74) Representative: **De Clercq & Partners**
**Edgard Gevaertdreef 10a**
**9830 Sint-Martens-Latem (BE)**

(56) References cited:
**EP-A1- 3 476 945** **WO-A1-2006/003721**
**WO-A2-2006/081284**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 575 415 B1

SEQUENCE LISTING

<110> Sysmex Corporation

<120> Method for synthesizing cDNA, method for detecting target RNA, and reagent kit

<130> SYSM-139-EP

<150> JP2018-103590
<151> 2018-05-30

<160> 16

<170> PatentIn version 3.5

<210> 1
<211> 23
<212> RNA
<213> Homo sapiens

<400> 1
uaagugcuuc cauguuuugg uga 23

<210> 2
<211> 34
<212> DNA
<213> Artificial Sequence

<220>
<223> primer

<400> 2
cgaggtattc gcactggata cgactcacca aaac 34

<210> 3
<211> 54
<212> DNA

<213> Artificial Sequence

<220>
<223> primer

<400> 3
gtcgtatcca gtgcagggtc cgaggtattc gcactggata cgactcacca aaac 54

<210> 4
<211> 24
<212> DNA
<213> Artificial Sequence

<220>
<223> second oligonucleotide molecule

<400> 4
gtcgtatcca gtgcgaatac ctcg 24

<210> 5
<211> 17
<212> DNA
<213> Artificial Sequence

<220>
<223> primer

<400> 5
taagtgcttc catgttt 17

<210> 6
<211> 13
<212> DNA
<213> Artificial Sequence

<220>
<223> primer

<400> 6
cgaggtattc gca 13

<210> 7
<211> 13
<212> DNA
<213> Artificial Sequence

<220>
<223> probe

<400> 7
atacgactca cca 13

<210> 8
<211> 21
<212> DNA
<213> Artificial Sequence

<220>

<223> second oligonucleotide molecule

<400> 8
gtcgtatcca gtgcgaatac c 21

<210> 9
<211> 18
<212> DNA
<213> Artificial Sequence

<220>
<223> second oligonucleotide molecule

<400> 9
gtcgtatcca gtgcgaat 18

<210> 10
<211> 24
<212> DNA
<213> Artificial Sequence

<220>
<223> second oligonucleotide molecule

<400> 10
gtcgtatcca ttgctaatac ctcg 24

<210> 11
<211> 24
<212> DNA
<213> Artificial Sequence

<220>
<223> second oligonucleotide molecule

<400> 11
gtcgtatcca ttactaatac ctcg 24

<210> 12
<211> 24
<212> DNA
<213> Artificial Sequence

<220>
<223> second oligonucleotide molecule

<400> 12
gttgtgttcg gtgtggatgc tttg 24

<210> 13
<211> 24
<212> DNA
<213> Artificial Sequence

<220>
<223> second oligonucleotide molecule

<400> 13

gttgtgtttg gtgtgggtgt tttg 24

<210> 14
<211> 37
<212> DNA
<213> Artificial Sequence

<220>
<223> primer

<400> 14
gtccgaggta ttcgcactgg atacgactca ccaaaac 37

<210> 15
<211> 27
<212> DNA
<213> Artificial Sequence

<220>
<223> second oligonucleotide molecule

<400> 15
gtcgtatcca gtgcgaatac ctcggac 27

<210> 16
<211> 17
<212> DNA
<213> Artificial Sequence

<220>
<223> primer

<400> 16
ccgaggtatt cgcactg 17

## Description

TECHNICAL FIELD

The present invention relates to a method for synthesizing cDNA. The present invention also relates to a method for detecting target RNA. Furthermore, the present invention relates to a reagent kit used for these methods.

BACKGROUND

In recent years, it has become apparent that small noncoding RNAs represented by microRNAs (miRNAs) play important roles in various biological processes such as development, differentiation, cell proliferation, and apoptosis. Detecting and quantifying such functional, small RNAs are very important in understanding biological phenomena. Generally, for detection of RNA, a method for synthesizing cDNA from RNA by reverse transcription reaction and amplifying and detecting the cDNA by PCR method is often used. However, since the nucleotide length of miRNA is as short as about 20 bases, it is difficult to design a primer that hybridizes to both ends of cDNA obtained by the reverse transcription reaction.

As a method for synthesizing and amplifying cDNA from miRNA, for example, the methods described in EP 1 851 336 B and US 7,575,863 are known. EP 1 851 336 B discloses that a reverse transcription reaction of miRNA is performed using a primer having a region that does not hybridize to miRNA on the 5' side to synthesize cDNA longer than the miRNA (see Fig. 1 of EP 1 851 336 B). US 7,575,863 discloses that a reverse transcription reaction of miRNA is performed using a stem-loop structure primer having a region that hybridizes to the miRNA as an overhang portion (see Fig. 4 of US 7,575,863). In the method of US 7,575,863, after the reverse transcription reaction, a stem portion of the primer is dissociated to obtain cDNA longer than the miRNA. The methods of EP 1 851 336 B and US 7,575,863 facilitate amplification of cDNA by PCR using the obtained cDNA as a template.

WO 2006/003721 A1 relates to the identification of nucleic acid molecules and cloning of fragments thereof. It discloses a double stranded linker that is used for reverse transcription of an RNA. The linker comprises a first and a second oligonucleotide, wherein a 3' overhanging region is complementary to the RNA. WO 2006/003721 A1 does not specifically relate to reverse transcription of small RNAs.

The present inventors have found that the methods of EP 1 851 336 B and US 7,575,863 leave room for improvement in sensitivity and specificity in the synthesis and amplification of cDNA from small RNAs. An object of the present invention is to develop a means capable of performing synthesis and amplification of cDNA from target RNA with higher accuracy.

SUMMARY

Accordingly, the present invention provides a method for synthesizing cDNA by mixing a target RNA, a first oligonucleotide molecule, a second oligonucleotide molecule, and a reverse transcriptase, using the target RNA as a template, wherein the target RNA is a small RNA selected from the group consisting of miRNA, snRNA, snoRNA, piRNA, pri-miRNA, pre-miRNA, siRNA, and shRNA. In the method, the first oligonucleotide molecule has a first region that hybridizes to the target RNA at the 3' end, and has a second region that hybridizes to the second oligonucleotide molecule on the 5' side (i.e. 5') of the first region.

According to the present invention, it is possible to perform synthesis and amplification of cDNA from target RNA with higher accuracy, wherein the target RNA is a small RNA selected from the group consisting of miRNA, snRNA, snoRNA, piRNA, pri-miRNA, pre-miRNA, siRNA, and shRNA.

BRIEF DESCRIPTION OF THE DRAWINGS

Fig. 1 is a schematic diagram showing a reaction principle in synthesis and amplification of cDNA by a synthesis method of an embodiment of the present invention.
Fig. 2A is a view showing an example of an appearance of a reagent kit according to an embodiment of the present invention.
Fig. 2B is a view showing an example of an appearance of a reagent kit according to an embodiment of the present invention.
Fig. 2C is a view showing an example of an appearance of a reagent kit according to an embodiment of the present invention.
Fig. 2D is a view showing an example of an appearance of a reagent kit according to an embodiment of the present

invention.

Fig. 3 is an amplification plot when cDNA synthesized by a method of EP 1 851 336 B is amplified by real-time PCR.

Fig. 4 is an amplification plot when cDNA synthesized by a method of US 7,575,863 is amplified by real-time PCR.

Fig. 5 is an amplification plot when cDNA synthesized by a method for synthesizing cDNA of an embodiment of the present invention is amplified by real-time PCR.

Fig. 6A is an amplification plot when cDNA synthesized by the method for synthesizing cDNA of an embodiment of the present invention using a second oligonucleotide molecule having a base sequence fully complementary to a base sequence of a second region of a first oligonucleotide molecule is amplified by real-time PCR.

Fig. 6B is an amplification plot when cDNA synthesized by the method for synthesizing cDNA of an embodiment of the present invention using a second oligonucleotide molecule having a base sequence containing a base that is not complementary to a base sequence of a second region of a first oligonucleotide molecule is amplified by real-time PCR.

Fig. 6C is an amplification plot when cDNA synthesized by the method for synthesizing cDNA of an embodiment of the present invention using a second oligonucleotide molecule having a base sequence containing a base that is not complementary to a base sequence of a second region of a first oligonucleotide molecule is amplified by real-time PCR.

Fig. 6D is an amplification plot when cDNA synthesized by the method for synthesizing cDNA of an embodiment of the present invention using a second oligonucleotide molecule having a base sequence containing a base that is not complementary to a base sequence of a second region of a first oligonucleotide molecule is amplified by real-time PCR.

Fig. 6E is an amplification plot when cDNA synthesized by the method for synthesizing cDNA of an embodiment of the present invention using a second oligonucleotide molecule having a base sequence containing a base that is not complementary to a base sequence of a second region of a first oligonucleotide molecule is amplified by real-time PCR.

Fig. 7A is an amplification plot when cDNA synthesized by the method for synthesizing cDNA of an embodiment of the present invention using a second oligonucleotide molecule into which a cap structure is introduced at 3' end is amplified by real-time PCR.

Fig. 7B is an amplification plot when cDNA synthesized by the method for synthesizing cDNA of an embodiment of the present invention using a second oligonucleotide molecule into which a cap structure is introduced at 3' end is amplified by real-time PCR.

DETAILED DESCRIPTION OF THE PREFERRED EMBODIMENTS

As used herein, the expression "hybridize" refers to that a whole or part of a predetermined oligonucleotide (or polynucleotide) forms a double strand via one or more hydrogen bonds with a whole or part of another oligonucleotide (or polynucleotide) under stringent conditions. The "stringent condition" may be a condition commonly used by a person skilled in the art when performing hybridization of oligonucleotides (or polynucleotides). Examples thereof include conditions in which one oligonucleotide molecule can specifically hybridize to the other oligonucleotide molecule when there is at least 50%, preferably at least 75%, and more preferably at least 90% sequence identity between the two oligonucleotide molecules. Stringency of hybridization is known to be a function of temperature, salt concentration, nucleotide length and GC content of the oligonucleotide and concentration of a chaotropic agent contained in a hybridization buffer. In particular embodiments, the stringent conditions, are the conditions described as stringent in, for example, Sambrook, J. et al., 1998, Molecular Cloning: A Laboratory Manual (2nd edition), Cold Spring Harbor Laboratory Press, New York.

As used herein, the term "base sequence fully complementary" refers to a base sequence that forms complementary base pairs according to the Watson-Crick model with all bases in the base sequence of a whole or part of a predetermined oligonucleotide (or polynucleotide). As used herein, the term "mismatch site" refers to a site in which complementary base pairs according to the Watson-Crick model cannot be formed because a base that is not complementary to a predetermined base in a base sequence of one oligonucleotide molecule (or polynucleotide molecule) is present at a corresponding position in a base sequence of the other oligonucleotide molecule (or polynucleotide molecule) when the two oligonucleotide molecules (or polynucleotide molecules) are hybridized. The meaning of the term "base sequence" equals to "nucleotide sequence."

As used herein, the term "reaction system" means a limited environment in which components necessary for reverse transcription reaction and/or nucleic acid amplification reaction are present and the reaction occurs. Examples of a reaction system for a reverse transcription reaction include tiny droplets such as a reaction solution or emulsion stored in a container such as a test tube, containing a target RNA, a first oligonucleotide molecule, a second oligonucleotide molecule, and a reverse transcriptase. The term "reverse transcriptase" is synonymous with "RNA-dependent DNA polymerase".

[1. Method for Synthesizing cDNA]

In the method for synthesizing cDNA of the present invention (hereinafter, also referred to as "synthesis method"), cDNA is synthesized by mixing a target RNA, a first oligonucleotide molecule, a second oligonucleotide molecule, and a reverse transcriptase, using the target RNA as a template, wherein the target RNA is a small RNA selected from the group consisting of miRNA, snRNA, snoRNA, piRNA, pri-miRNA, pre-miRNA, siRNA, and shRNA.

An example of a desirable reaction in the synthesis method of the present invention will be described with reference to Fig. 1. In this synthesis method, the first oligonucleotide molecule functions as a reverse transcription primer (hereinafter also referred to as "RT primer"). The first oligonucleotide molecule has a first region that hybridizes to the target RNA at the 3’ end, and has a second region that hybridizes to the second oligonucleotide molecule on the 5’ side of the first region. More particularly, the first oligonucleotide has a first region at its 3’ end and a second region at its 5’ end (which may or may not be separated by a further region). The first region of the first oligonucleotide molecule is capable of hybridizing to the target RNA, more particularly to the 3’ end of the target RNA. The second region of the first oligonucleotide molecule is capable of hybridizing to all or part of the second oligonucleotide molecule. When the target RNA, the first oligonucleotide molecule, the second oligonucleotide molecule, and the reverse transcriptase are mixed, as shown in the left side (1) of Fig. 1, the first region of the first oligonucleotide molecule hybridizes to the target RNA, and the second region hybridizes to the second oligonucleotide molecule. In the example of Fig. 1, the first region has a base sequence fully complementary to the base sequence of the portion containing the 3’ end of the target RNA. In this example, the second oligonucleotide molecule has the same nucleotide length as the second region, and has a base sequence fully complementary to the base sequence of the second region.

By placing the above mixture under temperature conditions suitable for reverse transcription reactions, as shown in the left side (2) of Fig. 1, the 3’ end of the first oligonucleotide molecule hybridized with the target RNA is extended to synthesize cDNA, by action of reverse transcriptase. Although cDNA is strictly a part of a complementary strand of target RNA, cDNA as used herein refers to an oligonucleotide containing not only the complementary strand of target RNA but also the second region of the first oligonucleotide molecule. That is, as used herein, cDNA refers to a first oligonucleotide molecule extended using a target RNA as a template. While the synthesized cDNA may hybridize to the target RNA and/or the second oligonucleotide molecule, the target RNA and the second oligonucleotide molecule can be easily removed by heat denaturation or the like.

As shown in Fig. 1, since the second region of the first oligonucleotide molecule is a region which does not hybridize to the target RNA, cDNA longer than the target RNA can be obtained. This facilitates design of primers used for cDNA amplification to be performed later. Although the synthesis method of the present invention is not limited by theory or action mechanism, it is presumed that extending the 3’ end of the first oligonucleotide molecule in a state in which a double strand of the second region of the first oligonucleotide molecule and the second oligonucleotide molecule is formed contributes to improvement in accuracy and specificity of cDNA synthesis and subsequent cDNA amplification.

In particular embodiments of the synthesis method of the present invention, the obtained cDNA may be amplified by a DNA amplification method such as PCR method. In this case, the mixture obtained after the reverse transcription reaction containing cDNA can be used as a template as it is. In the example of Fig. 1, cDNA is amplified by normal PCR method using a primer that hybridizes to the cDNA (hereinafter referred to as "forward primer"), a primer that hybridizes to a complementary strand of the cDNA (hereinafter referred to as "reverse primer") and a polymerase. In a first cycle of PCR, the forward primer hybridizes to cDNA as shown in the right side (3) of Fig. 1. Then, the forward primer is extended by action of polymerase to synthesize a complementary strand of the cDNA.

In a second cycle, the forward primer hybridizes to the cDNA and the reverse primer hybridizes to a complementary strand of the cDNA, as shown in the right side (4) of Fig. 1. Then, each primer is extended by the action of polymerase to obtain double stranded DNA. When the mixture after reverse transcription reaction is used as it is for PCR, in the example of Fig. 1, the reverse primer can also hybridize to the second oligonucleotide molecule remaining in the mixture. However, when a sufficiently larger amount of reverse primer than the second oligonucleotide molecule is used, it has little effect on the amplification reaction. In the third and subsequent cycles, as shown in the right side (5) of Fig. 1, amplification products are exponentially obtained using double-stranded DNA as a template.

Hereinafter, the components used for the synthesis method of the present invention will be described.

The target RNA is a small RNA selected from the group consisting of miRNA, snRNA, snoRNA, piRNA, pri-miRNA, pre-miRNA, siRNA, and shRNA and is not particularly limited, and can be appropriately selected from any RNA for which synthesis of cDNA is desired. In particular embodiments, in order to prepare a first oligonucleotide molecule as an RT primer, the target RNA is preferably RNA of which the base sequence is known. Information on the base sequences of RNA can be obtained from databases known in the art, for example, GenBank (http://www.ncbi.nlm.nih.gov/genbank/), miRBase (http://www.mirbase.org/search.shtml), and the like.

The synthesis method of the present invention is for cDNA synthesis from small RNAs. Small RNA refers to RNA, preferably non-coding RNA with a nucleotide length of less than 200. Small RNA is any one selected from the group consisting of miRNA, small nuclear RNA (snRNA), small nucleolar RNA (snoRNA), piwi-interacting RNA (piRNA),

primary miRNA (pri-miRNA), precursor miRNA (pre-miRNA), short interfering RNA (siRN) and short hairpin RNA (shRNA). In a preferred embodiment, the target RNA is a single stranded small RNA selected from the group consisting of miRNA, snRNA, snoRNA, piRNA, pri-miRNA, pre-miRNA, siRNA, and shRNA, having a nucleotide length of 15 or more and less than 200. In particular embodiments, the target RNA is less than 100, more particularly less than 50 nucleotides in length. In further embodiments, the target polynucleotides is between 15 and 40 nucleotides in length.

Although the source of the target RNA is not particularly limited, it may be RNA obtained from an organism or a biological sample, or may be artificially synthesized RNA. The organism is not particularly limited as long as it has the target RNA, and may be eukaryote or prokaryote. Preferred organisms are mammals including humans. Examples of the biological sample include organs, tissues, cells, body fluids and the like collected from an organism. Examples of the body fluid include blood, plasma, serum, lymph, saliva, urine, and the like. The biological sample may be cultured cells, cultures of microorganisms such as bacteria and viruses, culture supernatants, lysates, extracts, and the like. In recent years, it is known that exosomes contain a large amount of small RNAs such as miRNA. In a particular embodiment, the biological sample may be exosomes isolated from a body fluid or the like of an organism.

Methods for extracting RNA from a biological sample are publicly known per se. For example, when the biological sample is a cell or a tissue, extraction of RNA can be performed as follows. First, the biological sample is mixed with a solubilizing solution containing guanidine thiocyanate and a surfactant. Physical treatment (stirring, homogenizing, ultrasonic disruption, etc.) is performed on the resulting mixed solution to release RNA contained in the biological sample into the mixed solution. Thus, RNA can be extracted from the biological sample. The extracted RNA may be purified. For example, RNA can be purified by centrifuging a mixed solution containing RNA to collect a supernatant, and extracting the supernatant with phenol/chloroform. Extraction and purification of RNA from the biological sample may be performed using a commercially available RNA extraction kit.

The first oligonucleotide molecule is an oligonucleotide molecule having a first region at the 3' end and a second region on the 5' side of the first region. As described above, the first oligonucleotide molecule plays the role of an RT primer in the synthesis method of the present invention. The first region is a region that hybridizes to the target RNA, and the second region is a region that hybridizes to the second oligonucleotide molecule. In particular embodiments, the first oligonucleotide molecule may have another region in addition to the first region and the second region as long as extension of the 3' end by reverse transcription reaction is possible. In a preferred embodiment, the first oligonucleotide molecule includes only the first region and the second region, whereby the first region is directly adjacent to the second region. The first region and the second region of the first oligonucleotide molecule will be described below.

The first region is a region containing the 3' end of the first oligonucleotide molecule. The first region of the first oligonucleotide molecule hybridizes to the target RNA, and functions as an RT primer by having the 3' end extended. The nucleotide length of the first region is not particularly limited as long as it can maintain hybridization with the target RNA during the reverse transcription reaction. The first region has, for example, a nucleotide length of 3 or more and 15 or less, preferably 4 or more and 12 or less, and more preferably 6 or more and 10 or less. The base sequence of the first region is not particularly limited as long as it is a base sequence capable of hybridizing to the target RNA. Preferably, the base sequence of the first region is a base sequence fully complementary to a base sequence of a part of the target RNA. The portion of the target RNA to which the first region hybridizes is not particularly limited, and is preferably a portion containing the 3' end of the target RNA. In particular embodiments, the portion of the target RNA corresponds to less than 50% of the total length of the target RNA, and may also be less than 40 or about 30% of the total length of the target RNA.

The second region of the first oligonucleotide molecule is a region located on the 5' side of the first region. The second region may be a region containing the 5' end of the first oligonucleotide molecule. In particular embodiments, the second region does not comprise the 5' end of the first oligonucleotide molecule. As described above, the second region of the first oligonucleotide molecule hybridizes to the second oligonucleotide molecule. In particular embodiments, the whole of the second region may hybridize to the second oligonucleotide molecule. Alternatively, a part of the second region may hybridize to the second oligonucleotide molecule. Therefore, the whole or part of the second region has a base sequence capable of hybridizing to the second oligonucleotide molecule. Preferably, the base sequence of the whole or part of the second region is a base sequence fully complementary to an entire base sequence of the second oligonucleotide molecule. The nucleotide length of the second region is not particularly limited, and is, for example, a nucleotide length of 10 or more and 40 or less, preferably 17 or more and 40 or less, and more preferably 20 or more and 40 or less.

In the synthesis method provided herein, the second region is an additional sequence that imparts a length to a complementary strand of target RNA synthesized by the reverse transcription reaction. Therefore, the second region is preferably a region that hybridizes to the second oligonucleotide molecule but not to the target RNA. The base sequence of such second region can be appropriately determined according to the base sequence of the target RNA. The base sequence of the second region may also affect the efficiency of the subsequent amplification of cDNA, so that it is preferable to determine the base sequence in consideration of Tm value, GC content, and the like.

The first oligonucleotide molecule itself can be prepared by oligonucleotide synthesis methods known in the

art. The first oligonucleotide molecule may contain conventionally known artificial nucleic acids such as bridged nucleic acid (BNA), phosphorothioate (PS)-oligo, peptide nucleic acid (PNA), morpholino oligos and 2'-O-substituted RNAs as long as extension of the 3' end by the reverse transcription reaction is possible.

The second oligonucleotide molecule is an oligonucleotide molecule that hybridizes to a whole or part of the second region of the first oligonucleotide molecule. In particular embodiments, it is preferred that the second oligonucleotide molecule does not hybridize to the first region of the first oligonucleotide molecule. Although the synthesis method provided herein is not limited by theory or action mechanism, it is presumed that the second oligonucleotide molecule hybridizes to the second region of the first oligonucleotide molecule, thereby playing a role in preventing unexpected hybridization and nonspecific binding of the target polynucleotide to the second region.

The nucleotide length of the second oligonucleotide molecule may be the same length as the second region of the first oligonucleotide molecule or may be shorter than the second region. For example, the second oligonucleotide molecule has a nucleotide length of between 35% or more and 100% or less, preferably between 35% or more and 95% or less, and more preferably between 35% or more and 90% or less of the nucleotide length of the second region of the first oligonucleotide molecule.

When the second oligonucleotide molecule has a nucleotide length shorter than the second region of the first oligonucleotide molecule, a plurality of second oligonucleotide molecules, preferably two or three second oligonucleotide molecules may be used. Each second oligonucleotide molecule hybridizes to a different portion in the second region. The portions in the second region to which each second oligonucleotide molecule hybridizes may be adjacent or separated.

The base sequence of the second oligonucleotide molecule can be appropriately determined according to the base sequence of the second region of the first oligonucleotide molecule. The base sequence of the second oligonucleotide molecule may be a base sequence fully complementary to a whole or part of the second region of the first oligonucleotide molecule. Alternatively, the base sequence of the second oligonucleotide molecule may contain a base forming a mismatch site when hybridized with the second region of the first oligonucleotide molecule (hereinafter also referred to as "mismatched base"). For example, the ratio of the number of mismatched bases in the second oligonucleotide molecule (hereinafter also referred to as "mismatch rate") is 50% or less, preferably 40% or less, and more preferably 12% or less of its entire length. The mismatch rate is calculated by the following equation.

$$\text{(Mismatch rate)} = \{\text{(Number of mismatched bases in second oligonucleotide molecule)/(Nucleotide length of second oligonucleotide molecule)}\} \times 100$$

When the second oligonucleotide molecule contains a mismatched base, the second oligonucleotide molecule has a nucleotide length of preferably 35% or more and more preferably 100% of the nucleotide length of the second region of the first oligonucleotide molecule. More particularly, the each of the matched or mismatched bases in the second oligonucleotide molecule correspond to matched or mismatched bases in the second region of the first oligonucleotide molecule.

The second oligonucleotide molecule may contain uracil in the base sequence. When the synthesis method provided herein is performed using a second oligonucleotide molecule containing uracil, a part or whole of the second oligonucleotide molecule hybridized with the synthesized cDNA can be decomposed and removed by uracil DNA glycosylase (UNG).

The second oligonucleotide molecule itself can be prepared by oligonucleotide synthesis methods known in the art. The second oligonucleotide molecule may contain conventionally known artificial nucleic acids such as BNA, PS-oligo, PNA, morpholino oligo, and 2'-O-substituted RNA.

In particular embodiments, the 3' end of the second oligonucleotide molecule may be modified so as not to extend. Examples of such modification include phosphorylation, biotinylation, and the like. The 3' end can also be prevented from extending by introducing a modifying group such as dideoxyribonucleotide or an amino linker at the 3' end of the second oligonucleotide molecule.

In particular embodiments, the second oligonucleotide molecule may be modified to improve stability of binding to the second region. Examples of such modification include introduction of a cap structure at 5' end or 3' end. Examples of the cap structure introduced at the 5' end include a pyrene group, a trimethoxystilbene group, and the like. Examples of the cap structure introduced at the 3' end include a 2'-(anthraquinon-2-yl-carboxamide)-2'-deoxyuridine group (hereinafter also referred to as "Uaq") and the like. It is known that binding to the complementary strand is stabilized since the introduction of the cap structure improves the Tm value.

The reverse transcriptase is not particularly limited, and can be appropriately selected from known reverse transcriptases. Examples of the reverse transcriptase include avian myeloblastosis virus (AMV) reverse transcriptase,

Moloney murine leukemia virus (MMLV) reverse transcriptase, and the like. An enzyme contained in a commercially available reverse transcription reaction kit may be used.

In particular embodiments, for example, the target RNA, the first oligonucleotide molecule, the second oligonucleotide molecule and the reverse transcriptase are mixed in water (preferably nuclease-free water), and the obtained mixture (hereinafter, also referred to as "composition for RT") is placed under temperature conditions suitable for reverse transcription reactions, whereby cDNA is synthesized. The composition for RT may be further added with reagents used for general reverse transcription reaction such as buffer solutions (for example, Tris-HCl, etc.), dNTPs (mixtures of dATP, dCTP, dGTP and dTTP), inorganic salts (for example, NaCl, KCl, etc.), and RNase inhibitors. Such reagents are also included in commercially available reverse transcription reaction kits. The order in which the above components are mixed is not particularly limited. In particular embodiments, the first oligonucleotide molecule and the second oligonucleotide molecule may be mixed beforehand and used. However, it is not necessary to hybridize the first oligonucleotide molecule and the second oligonucleotide molecule beforehand. These oligonucleotide molecules spontaneously hybridize under temperature conditions suitable for reverse transcription reactions.

The addition amount of the first oligonucleotide molecule and the second oligonucleotide molecule is not particularly limited. The final concentrations of the first oligonucleotide molecule and the second oligonucleotide molecule in the composition for RT can be each appropriately determined from the range of 10 nM or more and 1000 nM or less, and preferably 10 nM or more and 250 nM or less. The final concentration of the second oligonucleotide molecule in the composition for RT may be the same as or different from the final concentration of the first oligonucleotide molecule.

The temperature conditions in the synthesis method provided herein are not particularly different from the conditions for the normal reverse transcription reaction. Depending on the type of reverse transcriptase to be used, the temperature conditions can be appropriately selected from known temperature conditions for reverse transcription reaction. In particular embodiments, a reverse transcription reaction can be performed using a commercially available thermal cycler.

In particular embodiments, the second oligonucleotide molecule may hybridize with the synthesized cDNA during the reaction. In particular embodiments, the synthesis method may include the step of removing at least a portion of the second oligonucleotide molecule after the synthesis of cDNA. For example, when the composition for RT after reverse transcription reaction is heated at 90°C to 99°C, the second oligonucleotide molecule is dissociated from cDNA by heat denaturation. When the second oligonucleotide molecule contains uracil, at least a portion of the second oligonucleotide molecule can be decomposed by UNG after the synthesis of cDNA. For example, uracil of the second oligonucleotide molecule hybridized with cDNA is removed by adding UNG to the composition for RT after reverse transcription reaction and incubating at a predetermined temperature (for example, 25°C). Since a portion that no longer contains uracil is structurally unstable, the second oligonucleotide molecule is cleaved at the portion that no longer contains uracil when the composition for RT is heated at 90°C to 99°C. This decomposes a part or whole of the second oligonucleotide molecule.

In the particular embodiments, in the same reaction system, different cDNAs may be synthesized using two or more types of target RNAs having different base sequences as a template. In this case, multiplex reverse transcription reaction can be performed by using a plurality of first oligonucleotide molecules having a first region according to the base sequence of each target RNA. The base sequence of the second region may be the same among the plurality of first oligonucleotide molecules or may be different from each other. The second oligonucleotide molecule can be appropriately designed according to the base sequence of the second region of the first oligonucleotide molecule.

The synthesis method provided herein may further include the step of amplifying cDNA. Amplification of cDNA itself can be performed by a DNA amplification method known in the art such as PCR method, and real-time PCR method. Amplification of cDNA is performed, for example, by mixing cDNA, a forward primer that hybridizes to the cDNA, a reverse primer that hybridizes to a complementary strand of the cDNA, and a polymerase. Since the first oligonucleotide molecule can also hybridize to the complementary strand of cDNA, the first oligonucleotide molecule may be used in place of the reverse primer for amplification of cDNA.

The forward primer and the reverse primer can be appropriately designed based on the base sequence of cDNA, i.e., the base sequences of the target RNA and the second region of the first oligonucleotide molecule. The nucleotide length of each primer is usually 5 or more and 50 or less, and preferably 10 or more and 40 or less. The primers themselves can be prepared by oligonucleotide synthesis methods known in the art.

The addition amount of the forward primer and the reverse primer is not particularly limited. The final concentrations of the forward primer and the reverse primer in a composition for PCR can be each appropriately determined from the range of 0.1 μM or more and 1.5 μM or less, and preferably 0.35 μM or more and 1.5 μM or less. The final concentration of the reverse primer in the composition for PCR may be the same as or different from the final concentration of the forward primer.

The forward primer and the reverse primer may contain conventionally known artificial nucleic acids such as BNA, PS-oligo, PNA, morpholino oligo, and 2'-O-substituted RNA. The forward primer and the reverse primer may be labeled with a known labeling substance. Examples of the labeling substance include radioactive isotopes (for example,

$^{32}P$, $^{35}S$, $^{3}H$, $^{14}C$, etc.), fluorescent dyes (for example, FITC, Texas Red (trademark), Alexa Fluor (trademark), 6-FAM, TAMRA, etc.), biotin, digoxigenin, and the like.

The polymerase is not particularly limited as long as it is a polymerase suitable for DNA amplification. Such a polymerase can be appropriately selected from known heat resistant DNA polymerases, and examples include Taq, Pfu, Tth, KOD, and the like. The polymerase contained in a commercially available PCR kit may be used.

In particular embodiments, cDNA, a forward primer, a reverse primer and/or a first oligonucleotide molecule and a polymerase are mixed in water, and the obtained mixture (hereinafter, also referred to as "composition for PCR") is placed under temperature conditions suitable for DNA amplification, whereby double-stranded DNA is obtained using cDNA as a template. The composition for PCR may be further added with reagents used for general DNA amplification reactions such as buffer solutions (for example, Tris-HCl, etc.), dNTPs, and inorganic salts (for example, NaCl, KCl, etc.). Such reagents are also included in commercially available PCR kits and the like. The order of mixing each of the above components is not particularly limited. In particular embodiments, the forward primer and the reverse primer and/or the first oligonucleotide molecule may be previously mixed and used.

When cDNA is amplified by real-time PCR, the composition for PCR may further comprise a fluorescent intercalator (for example, SYBR (trademark) Green, etc.), and a fluorescently labeled probe modified with a fluorescent substance at 5' end and with a quencher substance (for example, TaqMan (trademark) probe, etc.) at 3' end.

The temperature conditions for amplification of cDNA are not particularly different from the conditions for normal PCR method or real-time PCR method. Depending on the type of polymerase to be used, the Tm value of the primer and the like, the temperature conditions can be appropriately selected from known temperature conditions for DNA amplification reaction. In particular embodiments, amplification of cDNA can be performed using a commercially available thermal cycler or real-time PCR device.

In particular embodiments, the composition for RT after reverse transcription reaction may be used as it is as a template cDNA. While the cDNA in the composition may hybridize to the second oligonucleotide molecule, the second oligonucleotide molecule is dissociated from cDNA by a heat denaturation step in DNA amplification. When the second oligonucleotide molecule contains uracil, a part or whole of the second oligonucleotide molecule hybridized with cDNA can be decomposed and removed by adding UNG to the composition for RT after reverse transcription reaction and incubating at a predetermined temperature (for example, 25°C) and then performing DNA amplification.

In particular embodiments, the cDNA synthesis step and the cDNA amplification step may be performed in the same reaction system. In this case, for example, the target RNA, the first oligonucleotide molecule, the second oligonucleotide molecule, the reverse transcriptase, the forward primer, the reverse primer, and the polymerase are mixed. The obtained mixture is first placed under temperature conditions suitable for reverse transcription reactions to perform cDNA synthesis. Subsequently, the mixture is placed under temperature conditions suitable for a DNA amplification reaction to perform cDNA amplification. It is not necessary to transfer the mixture to another container between the reverse transcription reaction and the cDNA amplification reaction. Thus, the reverse transcription reaction and the cDNA amplification reaction can be performed in the same reaction system. In particular embodiments, the reverse transcription reaction and the cDNA amplification are performed subsequently in the same container. In place of the reverse transcriptase and the polymerase, an enzyme having both reverse transcription activity and DNA polymerase activity (for example, Tth, etc.) may be used. The above mixture may be further added with reagents used for general reverse transcription reaction and DNA amplification reaction such as buffer solutions (for example, Tris-HCl, etc.), dNTPs, inorganic salts (for example, NaCl, KCl, etc.), and RNase inhibitors.

[2. Method for Detecting Target RNA]

The scope of the present disclosure also includes a method for detecting target RNA (hereinafter also referred to as "detection method"), wherein the target RNA is a small RNA selected from the group consisting of miRNA, snRNA, snoRNA, piRNA, pri-miRNA, pre-miRNA, siRNA, and shRNA. In the detection method provided herein, first, a target RNA (or an RNA believed to potentially comprise the target RNA), a first oligonucleotide molecule, a second oligonucleotide molecule, and a reverse transcriptase are mixed, and cDNA is synthesized using the target RNA as a template. Details of the components used for cDNA synthesis (reverse transcription reaction), conditions for the reverse transcription reaction and the like are the same as those described for the synthesis method above. The method can comprise, in addition to carrying out the method with an RNA believed to potentially comprise the target RNA, carrying out the method with a control RNA which is known to comprise and/or a control known not to comprise the target RNA, as a positive and negative control, respectively.

Next, in the detection method provided herein, the synthesized cDNA is amplified, and an amplification product of the cDNA is detected. Details of the components used for cDNA amplification, conditions for the amplification reaction and the like are the same as those described for the synthesis method above. As used herein, the term "detecting" includes qualitatively determining the presence or absence of an amplification product, quantifying the amplification product, and detecting semiquantitatively the abundance of the amplification product. Semi-quantitative detection refers

that the abundance of the amplification product is indicated stepwise like "-", "+", "++" (negative, weak positive, strong positive), and the like. For example, when a result that an amplification product has been detected is obtained, the result indicates the presence of target RNA.

A means for detecting an amplification product is not particularly limited, and can be appropriately selected from known methods. For example, an amplification product may be detected by electrophoresis. Specifically, a reaction solution after the amplification reaction is electrophoresed on an agarose gel containing ethidium bromide to confirm the presence or absence of the amplification product, and the amount of the amplification product, if any. The amplification product may be detected by acquiring optical information such as fluorescence intensity, turbidity, and absorbance, from the reaction solution after the amplification reaction. For example, the amplification product may be detected by measuring the fluorescence intensity by an intercalator method using a fluorescent substance capable of binding to double-stranded DNA such as SYBR (trademark) Green.

Alternatively, the amplification product may be detected by detecting fluorescence generated from the probe by a method using the fluorescently labeled probe (for example, TaqMan (trademark) probe, etc.) modified with a fluorescent substance at the 5' end and with a quencher substance at the 3' end. Such a probe is designed to hybridize to a region different from the region to which the above forward primer and/or reverse primer hybridize in the amplification product to be detected. The final concentration of the fluorescently labeled probe in the composition for PCR is not particularly limited, and can be appropriately determined, for example, from the range of 0.1 $\mu$M or more and 5 $\mu$M or less, and preferably 0.2 $\mu$M or more and 0.8 $\mu$M or less.

[3. Reagent Kit]

The scope of the present disclosure also includes a reagent kit. This reagent kit can be used to perform the synthesis method provided herein and the detection method provided herein. The reagent kit includes a first oligonucleotide molecule that hybridizes to a target RNA, wherein the target RNA is a small RNA selected from the group consisting of miRNA, snRNA, snoRNA, piRNA, pri-miRNA, pre-miRNA, siRNA, and shRNA, a second oligonucleotide molecule that hybridizes to the first oligonucleotide molecule and a reverse transcriptase. Details of the first oligonucleotide molecule and the second oligonucleotide molecule are the same as those described for the synthesis method described above.

Containers respectively containing the first oligonucleotide molecule and the second oligonucleotide molecule may be contained in a box and provided to the user. The box may include all the above containers, or may contain some containers. The box may include an attached document describing a method of using the first oligonucleotide molecule and the second oligonucleotide molecule and the like. Fig. 2A shows an example of the reagent kit of the present invention. In the figure, 10 denotes a reagent kit, 11 denotes a first container containing a first oligonucleotide molecule, 12 denotes a second container containing a second oligonucleotide molecule, 13 denotes a packing box, and 14 denotes the attached document.

The concentration of the first oligonucleotide molecule in the first container may be a concentration that can be adjusted to the above final concentration when used for preparation of the composition for RT. Therefore, the concentration of the first oligonucleotide molecule in the container can be appropriately determined from a volume ratio of a total amount of the composition for RT and an addition amount of the first oligonucleotide molecule. The concentration of the first oligonucleotide molecule in the first container can be, for example, 0.05 $\mu$M or more and 100 $\mu$M or less, and preferably 0.25 $\mu$M or more and 10 $\mu$M or less.

The concentration of the second oligonucleotide molecule in the second container can also be determined similar to the concentration of the first oligonucleotide molecule. The concentration of the second oligonucleotide molecule in the second container can be, for example, 0.05 $\mu$M or more and 100 $\mu$M or less, and preferably 0.25 $\mu$M or more and 10 $\mu$M or less. The concentration of the second oligonucleotide molecule in the second container may be the same as or different from the concentration of the first oligonucleotide molecule in the first container.

In particular embodiments, the reagent kit may further include a reverse transcriptase. The reagent kit may further include dNTP. The reverse transcriptase itself is known in the art, and can be appropriately selected from, for example, enzymes derived from retrovirus such as AMV and MMLV. The dNTP may be a mixture of dATP, dCTP, dGTP and dTTP. Fig. 2B shows an example of a reagent kit further including a reverse transcriptase and dNTP. In the figure, 20 denotes a reagent kit, 21 denotes a first container containing a first oligonucleotide molecule, 22 denotes a second container containing a second oligonucleotide molecule, 23 denotes a third container containing a reverse transcriptase, 24 denotes a fourth container containing dNTP, 25 denotes a packing box, and 26 denotes an attached document. Although not shown, the reagent kit may further include a buffer solution suitable for reverse transcription reactions.

In particular embodiments, the second oligonucleotide contains at least one uracil. In further particular embodiments, the kit comprises UNG.

In particular embodiments, the reagent kit may further include a forward primer that hybridizes to cDNA synthesized by extension of the first oligonucleotide molecule, and a reverse primer that hybridizes to a complementary

strand of the cDNA. Details of the forward primer and the reverse primer are the same as those described for the synthesis method described above. Fig. 2C shows an example of a reagent kit further including a forward primer and a reverse primer. In the figure, 30 denotes a reagent kit, 31 denotes a first container containing a first oligonucleotide molecule, 32 denotes a second container containing a second oligonucleotide molecule, 33 denotes a third container containing a reverse transcriptase, 34 denotes a fourth container containing dNTP, 35 denotes a fifth container containing a forward primer, 36 denotes a sixth container containing a reverse primer, 37 denotes a packing box, and 38 denotes an attached document. Although not shown, in particular embodiments, the reagent kit may further include a buffer solution suitable for reverse transcription reactions.

The concentration of the forward primer in the fifth container may be any concentration that can be adjusted to the above final concentration when used for preparation of the composition for PCR. Therefore, the concentration of the forward primer in the container can be appropriately determined from a volume ratio of the total amount of the composition for PCR and an addition amount of the forward primer. The concentration of the forward primer in the fifth container can be, for example, 6 $\mu$M or more and 100 $\mu$M or less, and preferably 15 $\mu$M or more and 100 $\mu$M or less.

The concentration of the reverse primer in the sixth container can also be determined similar to the concentration of the forward primer. The concentration of the reverse primer in the sixth container can be, for example, 3 $\mu$M or more and 100 $\mu$M or less, and preferably 15 $\mu$M or more and 100 $\mu$M or less. The concentration of the reverse primer in the sixth container may be the same as or different from the concentration of the forward primer in the fifth container.

The reagent kit may further include a polymerase. The reagent kit may further include a fluorescently labeled probe. Details of the polymerase and the fluorescently labeled probe are the same as those described for the synthesis method and detection method described above. Fig. 2D shows an example of a reagent kit further including a polymerase and a fluorescently labeled probe. In the figure, 40 denotes a reagent kit, 41 denotes a first container containing a first oligonucleotide molecule, 42 denotes a second container containing a second oligonucleotide molecule, 43 denotes a third container containing a reverse transcriptase, 44 denotes a fourth container containing dNTP, 45 denotes a fifth container containing a forward primer, 46 denotes a sixth container containing a reverse primer, 47 denotes a seventh container containing a polymerase, 48 denotes an eighth container containing a fluorescently labeled probe, 49 denotes a packing box, and 50 denotes an attached document. Although not shown, the reagent kit may further include a buffer solution suitable for reverse transcription reactions and a buffer solution suitable for DNA amplification reactions.

The concentration of the fluorescently labeled probe in the eighth container may be any concentration that can be adjusted to the above final concentration when used for the preparation of the composition for PCR. Therefore, the concentration of the fluorescently labeled probe in the container can be appropriately determined from a volume ratio of the total amount of the composition for PCR and an addition amount of the fluorescently labeled probe. The concentration of the fluorescently labeled probe in the eighth container can be, for example, 2 $\mu$M or more and 25 $\mu$M or less, and preferably 2 $\mu$M or more and 10 $\mu$M or less.

At least two of the first oligonucleotide, the second oligonucleotide, the forward primer and the reverse primer may be contained in the same container. Preferably, the first oligonucleotide and the second oligonucleotide are contained in the same container. Alternatively, the forward primer and the reverse primer are contained in the same container. More preferably, the first oligonucleotide and the second oligonucleotide are contained in the same container, and the forward primer and the reverse primer are contained in another same container.

[4. Oligonucleotide molecule and methods of production]

The scope of the present disclosure also includes oligonucleotide molecules that hybridizes to a target RNA, wherein the oligonucleotide molecule comprising a first region at 3' end and a second region on 5' side of the first region, the first region hybridizing to the target RNA, and the second region hybridizing to the oligonucleotide molecule and an oligonucleotide molecule different from the target RNA. In particular embodiments, the first oligonucleotide is DNA.

The scope of the present disclosure also includes a (partly) double stranded molecule comprising a target RNA, a first oligonucleotide and a second oligonucleotide as defined herein above. In particular embodiments, the first oligonucleotide comprises a DNA sequence which is complementary to part of the target RNA.

The scope of the present disclosure also includes methods of making oligonucleotide molecules suitable for use in the methods of the present invention, wherein the oligonucleotide molecules hybridize to a target RNA, wherein the oligonucleotide molecule comprises a first region at 3' end and a second region on 5' side of the first region, the first region hybridizing to the target RNA, and the second region hybridizing to the oligonucleotide molecule and an oligonucleotide molecule different from the target RNA. These methods comprise the steps of (a) identifying the target RNA and (b) developing an oligonucleotide molecule that hybridize to a target RNA, wherein the oligonucleotide molecule comprises a first region at 3' end and a second region on 5' side of the first region, the first region hybridizing to the target RNA, and the second region hybridizing to the oligonucleotide molecule and an oligonucleotide molecule different from the target RNA, and (c) synthesizing said oligonucleotide molecule. The oligonucleotide molecules that hybridize to the target RNA comprising a first and second region comprise the features of the first oligonucleotide molecule

described herein above. The oligonucleotide different from the target RNA has the features of the second oligonucleotide molecule as detailed herein above. The second region is preferably a region that hybridizes to the oligonucleotide different from the target molecule (cfr. second oligonucleotide molecule described above) but not to the target RNA. The base sequence of such second region can be appropriately determined according to the base sequence of the target RNA. The base sequence of the second region can be determined in consideration of Tm value, GC content, and the like, to further the efficiency of the subsequent amplification of cDNA in the methods of the invention.

The scope of the present disclosure also includes methods of producing a combination of first and second oligonucleotide molecules suitable for use in the methods of the invention as described herein.The methods include selecting suitable base sequences for the first and second regions for the first oligonucleotide molecule, determining a suitable corresponding base sequence for the second oligonucleotide molecule and optionally, adjusting said base sequence of said second region and said corresponding second oligonucleotide molecule in consideration of Tm value, GC content, and the like, to further the efficiency of the subsequent amplification of cDNA in the methods of the invention. The methods then also include the step of synthesizing said oligonucleotide molecule(s). Methods for oligonucleotide synthesis are known in the art. Said method may further comprises adjusting said base sequence of said second oligonucleotide molecule to include uracil, as detailed herein above.

Herein below, the present invention will be described in detail by examples, but the present invention is not limited to these examples.

## EXAMPLES

### Example 1

In Example 1, cDNA was synthesized from target RNA (miRNA) by the method of EP 1 851 336 B, the method of US 7,575,863, and the synthesis method of the present invention, and the cDNA was amplified and detected by real-time PCR. Then, the lower limit of detection and the lower limit of quantification of the amplification product were compared. In Example 1, the experiment was performed in triplicate.

(1) Synthesis of cDNA

(1.1) Target RNA and Reverse Transcription Primer

An oligonucleotide of a base sequence represented by SEQ ID NO: 1 was used as target RNA. This base sequence is a base sequence of miR302a. An oligonucleotide of a base sequence represented by SEQ ID NO: 2 was used as a reverse transcription primer (hereinafter, referred to as "RT primer"). This oligonucleotide is not only an RT primer used in the method of EP 1 851 336 B, but also a first oligonucleotide molecule used in the synthesis method of the present invention. In the base sequence represented by SEQ ID NO: 2, 1st to 24th base sequence counted from 5' side is a region (second region) which does not hybridize to the target RNA, and 25th to 34th base sequence is a region (first region) that hybridizes to the target RNA. An oligonucleotide of a base sequence represented by SEQ ID NO: 3 was used as a stem-loop RT primer used in the method of US 7,575,863. An oligonucleotide of a base sequence represented by SEQ ID NO: 4 was used as a second oligonucleotide molecule used in the synthesis method of the present invention. This base sequence is fully complementary to the 1st to 24th base sequences counted from the 5' side of the base sequence represented by SEQ ID NO: 2. The base sequences of the oligonucleotides are shown below. Underlined portions indicate regions where the target RNA hybridizes to each RT primer.

- Target RNA (miR302a): 5'- UAAGUGCUUCCAUGUUUUGGUGA -3' (SEQ ID NO: 1)
- RT Primer:5'- CGAGGTATTCGCACTGGATACGACTCACCAAAAC -3' (SEQ ID NO: 2)
- Stem-loop RT primer: 5'-GTCGTATCCAGTGCAGGGTCCGAGGTATTCGCACTGGATACGACTCACCAAA AC -3' (SEQ ID NO: 3)
- Second oligonucleotide molecule: 5'- GTCGTATCCAGTGCGAATACCTCG -3' (SEQ ID NO: 4)

(1.2) Reverse Transcription Reaction

A mixture of the following composition was prepared using the above oligonucleotide and TaqMan (trademark) MicroRNA Reverse Transcription Kit (Applied Biosystems, product number 4366597). The kit contained 100 mM dNTP, 10 × buffer, an RNase inhibitor and a reverse transcriptase. In the synthesis method of the present invention, the RT primer of SEQ ID NO: 2 and the second oligonucleotide molecule were previously mixed to prepare a 250 nM RT primer. The concentration of target RNA was adjusted with nuclease-free water to obtain solutions containing 500, $10^3$, $10^4$, $10^5$, $10^6$ or $10^7$ copies of miRNA per $\mu$L. In the synthesis method of the present invention and the method of EP 1 851

336 B, solutions containing $10^3$, $10^5$ or $10^7$ copies of miRNA per μL were used as target RNA. In the method of US 7,575,863, solutions containing 500, $10^3$, $10^4$, $10^5$, or $10^6$ copies of miRNA per μL were used as target RNA. A mixture was similarly prepared except that yeast tRNA was used in place of the target RNA to obtain a negative control.

<Composition per Well>

| | |
|---|---|
| Nuclease-free water | 8.16 μL |
| 100 mM dNTP | 0.15 μL |
| 10 × Buffer | 1.5 μL |
| RNase Inhibitor | 0.19 μL |
| Reverse transcriptase | 1 μL |
| 250 nM RT primer | 3 μL |
| Target RNA | 1 μL |
| Total | 15 μL |

The prepared mixture was set to GeneAmp PCR System 9700 (Applied Biosystems), and reverse transcription reaction was performed under temperature conditions of 16°C for 30 minutes, 42°C for 30 minutes, and 85°C for 5 minutes.

(2) Amplification and Detection of cDNA by Real-Time PCR

(2.1) Primer Set and Probe for PCR

Oligonucleotides of base sequences represented by SEQ ID NOS: 5, 6 and 7 were used as a forward primer, a reverse primer, and a probe, respectively. The base sequences of the oligonucleotides are shown below. The forward primer and the reverse primer were mixed to prepare a primer mix. The concentration of the forward primer in the primer mix was 30 μM, and the concentration of the reverse primer was 15 μM. The probe was a TaqMan (trademark) probe modified with a fluorescent substance at 5' end and with a quencher substance at 3' end.

- Forward primer: 5'- TAAGTGCTTCCATGTTT -3' (SEQ ID NO: 5)
- Reverse primer: 5'- CGAGGTATTCGCA -3' (SEQ ID NO: 6)
- TaqMan (trademark) Probe: 5'- ATACGACTCACCA -3' (SEQ ID NO: 7)

(2.2) Real-time PCR

A mixture of the following composition was prepared using the above reverse transcription product, the primer mix and the probe, and TaqMan (trademark) Universal Master Mix II, with UNG (Applied Biosystems, product number 4440038).

<Composition per Well>

| | |
|---|---|
| Primer mix | 1 μL |
| 8 μM Probe | 2 μL |
| Master mix II | 10 μL |
| Reverse transcript | 1.33 μL |
| Distilled water | 5.67 μL |
| Total | 20 μL |

The prepared mixture was set to 7500 Fast Real-Time PCR System (Applied Biosystems), and PCR was performed under the following temperature conditions. Heating and cooling were performed at a rate of 1.6°C/sec.

<Temperature Conditions>

At 95°C for 10 minutes,
40 cycles at 95°C for 15 seconds and at 55°C for 60 seconds.

(3) Analysis and Results

The amplification plots obtained by the method of EP 1 851 336 B, the method of US 7,575,863, and the synthesis method of the present invention are shown in Figs. 3 to 5, respectively. In these plots, the abscissa represents the cycle number, and the ordinate represents the change in fluorescence intensity (ΔRn). In the figures, "10^3", "10^5", "10^6" and "10^7" represent cDNA amplification products derived from specimens containing $10^3$, $10^5$, $10^6$ and $10^7$ copies of the target RNA, respectively. "NC" indicates a negative control. In the amplification plots obtained by the methods of EP 1 851 336 B and US 7,575,863, a threshold value of ΔRn was set to 0.1, and in the amplification plot obtained by the synthesis method of the present invention, a threshold value of ΔRn was set to 0.03. Then, Ct (Threshold Cycle) values were obtained from the points of contact with the respective amplification curves. The copy number of the target RNA in the mixture of (1.2) above was defined as an initial template amount, and a calibration curve was created from the Ct value and a logarithm of the initial template amount (not shown).

The lower limit of detection and the lower limit of quantification of each method were determined based on the calibration curve. The results are shown in Table 1. Lower limit of quantification (1) was determined from the range in which the value of correlation coefficient ($R^2$) of the calibration curve was larger than 0.98. Lower limit of quantification (2) was determined from the range in which the slope of the calibration curve was -3.91 or more and -2.92 or less (within amplification efficiency ± 20%). The lower limit of detection is a value at which a p value is smaller than 0.01 when T test (two-sided test) is performed on the Ct value of the negative control (yeast tRNA). As the T test, Excel (trademark) function T. TEST was used.

[Table 1]

| | Lower limit of quantification (1) | Lower limit of quantification (2) | Lower limit of detection |
|---|---|---|---|
| EP 1 851 336 B | $10^5$ | $10^5$ | $10^5$ |
| US 7,575,863 | $10^3$ | $10^5$ | $10^3$ |
| Present invention | $10^3$ | $10^3$ | $10^3$ |

As shown in Table 1, the synthesis method provided herein was found to have higher performance in detection and quantification of the amplification products than the methods of the prior art EP 1 851 336 B and US 7,575,863. As shown in Fig. 4, in the method of US 7,575,863, the negative control was also amplified, and nonspecific amplification was observed. On the other hand, as shown in Fig. 5, almost no nonspecific amplification was observed in the synthesis method of the present invention. Accordingly, it was shown that the synthesis method of the invention has higher sensitivity and specificity in synthesis and amplification of cDNA from small RNAs, as compared to the prior art.

Example 2

In Example 2, cDNA was synthesized from target RNA by the synthesis method provided herein using second oligonucleotide molecules of different nucleotide lengths, and the cDNA was amplified and detected by real-time PCR. Then, the lower limit of detection and the lower limit of quantification of the amplification product were compared. In Example 2, the experiment was performed in triplicate.

(1) Target RNA and Reverse Transcription Primer

The oligonucleotides of the base sequences represented by SEQ ID NOS: 1 and 2 were used as target RNA and RT primers, respectively. Oligonucleotides of the base sequences shown below were used as the second oligonucleotide molecules. Hereinafter, these second oligonucleotide molecules are also referred to as "Lid", "Lid-S 1", "Lid-S2" and "Lid-S3", respectively. The base sequences of each second oligonucleotide molecule are shown below. The ratios of the nucleotide length of each second oligonucleotide molecule to the nucleotide length (24 bases) of the second region of the RT primer are shown in Table 2.

- Lid: 5'- GTCGTATCCAGTGCGAATACCTCG -3' (SEQ ID NO: 4)
- Lid-S1: 5'- GTCGTATCCAGTGCGAATACC -3' (SEQ ID NO: 8)
- Lid-S2: 5'- GTCGTATCCAGTGCGAAT -3' (SEQ ID NO: 9)
- Lid-S3: 5'- GTCGTATCC -3'

[Table 2]

| | Nucleotide length | Ratio (%) to nucleotide length of second region |
|---|---|---|
| Lid | 24 | 100 |
| Lid-S1 | 21 | 87.5 |
| Lid-S2 | 18 | 75.0 |
| Lid-S3 | 9 | 37.5 |

(2) cDNA Synthesis, Amplification and Detection

Reverse transcription reaction was performed to synthesize cDNA in the same manner as in Example 1 except that the above second oligonucleotide molecule was used. The obtained cDNA was amplified and detected by real-time PCR in the same manner as in Example 1 to obtain an amplification plot (not shown).

(3) Analysis and Results

A calibration curve was created from each amplification plot, with a threshold value of ΔRn of 0.05. The lower limit of detection and the lower limit of quantification were determined in the same manner as in Example 1 based on the obtained calibration curve. The results are shown in Table 3.

[Table 3]

| | Lower limit of quantification (1) | Lower limit of quantification (2) | Lower limit of detection |
|---|---|---|---|
| Lid | $10^3$ | $10^3$ | $10^3$ |
| Lid-S1 | $10^3$ | $10^3$ | $10^3$ |
| Lid-S2 | $10^3$ | $10^3$ | $10^3$ |
| Lid-S3 | $10^3$ | $10^3$ | $10^3$ |

As shown in Table 3, it was found that performance of detection and quantification of the amplification products was high even using a second oligonucleotide molecule of any nucleotide length. From the amplification plot, almost no nonspecific amplification was observed even using a second oligonucleotide molecule of any nucleotide length.

Example 3

In Example 3, cDNA was synthesized from target RNA by the synthesis method provided herein using a second oligonucleotide molecule that forms a mismatch site when hybridized with the second region of the RT primer, and the cDNA was amplified and detected by real-time PCR. Then, the lower limit of detection and the lower limit of quantification of the amplification product were compared. In Example 3, the experiment was performed in triplicate.

(1) Target RNA and Reverse Transcription Primer

The oligonucleotides of the base sequences represented by SEQ ID NOS: 1 and 2 were used as target RNA and RT primers, respectively. Oligonucleotides of base sequences represented by SEQ ID NOS: 4 and 10 to 13 were used as the second oligonucleotide molecules. The oligonucleotides of the base sequences represented by SEQ ID NOS: 10 to 13 are also referred to as "Lid-M1", "Lid-M2", "Lid-M3" and "Lid-M4", respectively. The base sequences of each second oligonucleotide molecule are shown below. Underlined portions indicate mismatched bases in the second oligonucleotide molecules. The mismatch rate of the second oligonucleotide molecule was shown in Table 4.

- Lid: 5'- GTCGTATCCAGTGCGAATACCTCG -3' (SEQ ID NO: 4)
- Lid-M1: 5'- GTCGTATCCATTGCTAATACCTCG -3' (SEQ ID NO: 10)
- Lid-M2: 5'- GTCGTATCCATTACTAATACCTCG -3' (SEQ ID NO: 11)
- Lid-M3: 5'- GTTGTGTTCGGTGTGGATGCTTTG -3' (SEQ ID NO: 12)
- Lid-M4: 5'- GTTGTGTTTGGTGTGGGTGTTTTG -3' (SEQ ID NO: 13)

[Table 4]

| | Number of mismatched bases | Mismatch rate (%) |
|---|---|---|
| Lid | 0 | 0 |
| Lid-M1 | 2 | 8.3 |
| Lid-M2 | 3 | 12.5 |
| Lid-M3 | 9 | 37.5 |
| Lid-M4 | 12 | 50.0 |

(2) cDNA Synthesis, Amplification and Detection

Reverse transcription reaction was performed to synthesize cDNA in the same manner as in Example 1 except that the above second oligonucleotide molecule was used. As in Example 1, the obtained cDNA was amplified and detected by real-time PCR. The obtained amplification plots are shown in Figs. 6A to 6E.

(3) Analysis and Results

A calibration curve was created from each amplification plot, with a threshold value of $\Delta$Rn of 0.05. The lower limit of detection and the lower limit of quantification were determined in the same manner as in Example 1 based on the obtained calibration curve. The results are shown in Table 5.

[Table 5]

| | Lower limit of quantification (1) | Lower limit of quantification (2) | Lower limit of detection |
|---|---|---|---|
| Lid | $10^3$ | $10^3$ | $10^3$ |
| Lid-M1 | $10^3$ | $10^3$ | $10^3$ |
| Lid-M2 | $10^3$ | $10^3$ | $10^3$ |
| Lid-M3 | $10^3$ | $10^3$ | $10^3$ |
| Lid-M4 | $10^3$ | $10^3$ | $10^3$ |

As shown in Table 5, it was found that performance of detection and quantification of the amplification products was high even using the second oligonucleotide molecule having a mismatched base. From Fig. 6A to 6E, almost no nonspecific amplification was observed even when about 50% of mismatch was present between the second region of the RT primer and the second oligonucleotide molecule.

Example 4

In Example 4, cDNA was synthesized from target RNA by the synthesis method provided herein using a second oligonucleotide molecule having a CAP structure added at 3' end, and the cDNA was amplified and detected by real-time PCR. Then, the lower limit of detection and the lower limit of quantification of the amplification product were compared. In Example 4, the experiment was performed in triplicate.

(1) Target RNA and Reverse Transcription Primer

An oligonucleotide of a base sequence represented by SEQ ID NO: 1 was used as target RNA. The oligonucleotides of the base sequences represented by SEQ ID NOS: 2 and 14 were used as RT primers. Hereinafter, these RT primers are also referred to as "RT-primer 1" and "RT-primer 2", respectively. Oligonucleotides of base sequences represented by SEQ ID NOS: 4 and 15 were used as the second oligonucleotide molecules. In Example 4, Uaq was added as a cap structure at 3' ends of these oligonucleotides. The addition of 3'-Uaq CAP is expected to improve the Tm value and improve stability of binding to the complementary strand. Hereinafter, the second oligonucleotide molecules added with 3'-Uaq CAP are also referred to as "Lid-Capl" and "Lid-Cap2", respectively. A chemical structure of 3'-Uaq CAP is shown below. The base sequences of the RT primer and the second oligonucleotide molecule are shown below.

[Chemical Formula 1]

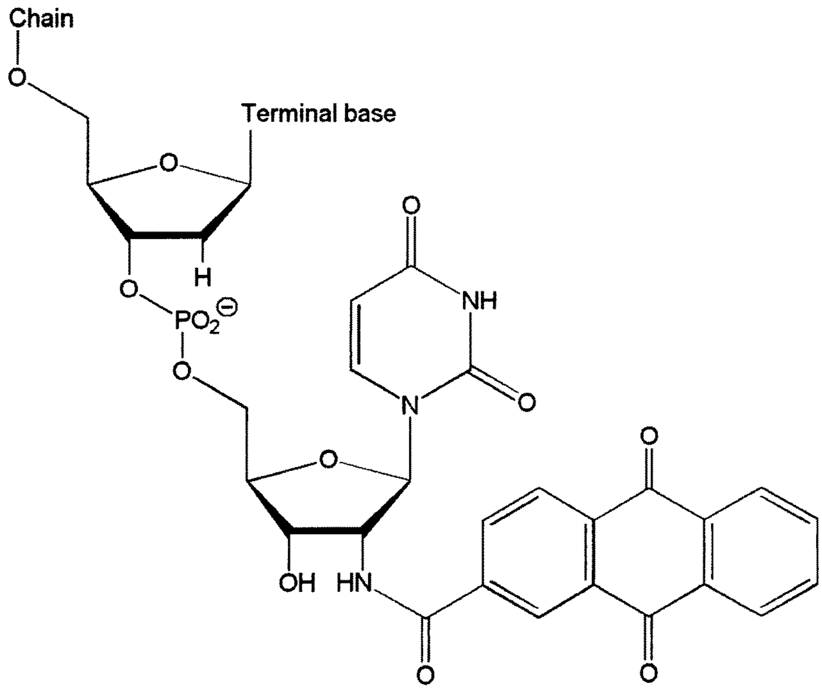

- RT-primer 1: 5'- CGAGGTATTCGCACTGGATACGACTCACCAAAAC -3' (SEQ ID NO: 2)
- RT-primer 2: 5'- GTCCGAGGTATTCGCACTGGATACGACTCACCAAAAC -3' (SEQ ID NO: 14)
- Lid: 5'- GTCGTATCCAGTGCGAATACCTCG -3' (SEQ ID NO: 4)
- Lid-Cap 1: 5'- GTCGTATCCAGTGCGAATACCTCG-Uaq -3' (SEQ ID NO: 4)
- Lid-Cap 2: 5'- GTCGTATCCAGTGCGAATACCTCGGAC-Uaq -3' (SEQ ID NO: 15)

(2) cDNA Synthesis, Amplification and Detection

Reverse transcription reaction was performed to synthesize cDNA in the same manner as in Example 1 except that the RT primer and the second oligonucleotide molecule were used. Lid or Lid-Cap 1 was used as a second oligonucleotide molecule for RT-primer 1, and Lid-Cap 2 was used as a second oligonucleotide molecule for RT-primer 2. As in Example 1, the obtained cDNA was amplified and detected by real-time PCR. In amplification of cDNA synthesized using RT-primer 2, in place of the reverse primer of the base sequence represented by SEQ ID NO: 6, a reverse primer of a base sequence represented by SEQ ID NO: 16 below was used. The amplification plots using Lid-Cap 1 and Lid-Cap 2 are shown in Figs. 7A and 7B, respectively.

- Reverse primer: 5'- CCGAGGTATTCGCACTG -3' (SEQ ID NO: 16)

(3) Analysis and Results

A calibration curve was created from each amplification plot, with a threshold value of ΔRn of 0.1. The lower

limit of detection and the lower limit of quantification were determined in the same manner as in Example 1 based on the obtained calibration curve. The results are shown in Table 6.

[Table 6]

| | Lower limit of quantification (1) | Lower limit of quantification (2) | Lower limit of detection |
|---|---|---|---|
| Lid | $10^3$ | $10^3$ | $10^3$ |
| Lid-Cap 1 | $10^3$ | $10^3$ | $10^3$ |
| Lid-Cap 2 | $10^3$ | $10^3$ | $10^3$ |

As shown in Table 6, it was found that performance of detection and quantification of the amplification products was high also when using the second oligonucleotide molecule having a CAP structure added at the 3' end, as well as when using an unmodified second oligonucleotide molecule. Figs. 7A and 7B show that nonspecific amplification is suppressed when using the second oligonucleotide molecule having a CAP structure added at the 3' end. Accordingly, it was suggested that the addition of the CAP structure to the 3' end of the second oligonucleotide molecule has an effect of suppressing nonspecific amplification.

## Claims

1. A method for synthesizing cDNA by mixing a target RNA, a first oligonucleotide molecule, a second oligonucleotide molecule, and a reverse transcriptase, using the target RNA as a template, the first oligonucleotide molecule comprising a first region that hybridizes to the target RNA at 3' end, and comprising a second region, 5' of the first region, that hybridizes to the second oligonucleotide molecule, wherein the target RNA is a small RNA selected from the group consisting of miRNA, snRNA, snoRNA, piRNA, pri-miRNA, pre-miRNA, siRNA, and shRNA.

2. The method according to claim 1, wherein
the first region of the first oligonucleotide molecule hybridizes to the target RNA, and the second region of the first oligonucleotide molecule hybridizes to the second oligonucleotide molecule, and
the reverse transcriptase extends the 3' end of the first oligonucleotide molecule hybridized with the target RNA and the second oligonucleotide molecule to synthesize cDNA.

3. The method according to claim 1 or 2, wherein a nucleotide length of the first region of the first oligonucleotide molecule is 3 or more and 15 or less.

4. The method according to any one of claims 1 to 3, wherein a nucleotide length of the second region of the first oligonucleotide molecule is 10 or more and 40 or less.

5. The method according to any one of claims 1 to 4, wherein a nucleotide length of the second oligonucleotide molecule is 35% or more and 100% or less of the nucleotide length of the second region of the first oligonucleotide molecule.

6. The method according to any one of claims 1 to 5, wherein a ratio of the number of bases forming a mismatch site when hybridized with the second region of the first oligonucleotide molecule in the second oligonucleotide molecule is within 50%.

7. The method according to any one of claims 1 to 6, wherein the 3' end of the second oligonucleotide molecule is modified so as not to extend.

8. The method according to any one of claims 1 to 7, further comprising a step of amplifying the cDNA.

9. The method according to claim 8, wherein the cDNA amplification step comprises mixing the cDNA, a forward primer that hybridizes to the cDNA, a reverse primer that hybridizes to a complementary strand of the cDNA and/or a first oligonucleotide molecule, and a polymerase.

10. The method according to any one of claims 1 to 9, further comprising the step of removing at least a portion of the second oligonucleotide molecule after the synthesis of cDNA.

11. A method for detecting target RNA, comprising the steps of:

synthesizing cDNA by mixing a target RNA, a first oligonucleotide molecule, a second oligonucleotide molecule, and a reverse transcriptase, using the target RNA as a template; and
amplifying the cDNA and detecting an amplification product,
the first oligonucleotide molecule comprising a first region that hybridizes to the target RNA at 3' end, and comprising a second region that hybridizes to the second oligonucleotide molecule on 5' side of the first region, wherein the target RNA is a small RNA selected from the group consisting of miRNA, snRNA, snoRNA, piRNA, pri-miRNA, pre-miRNA, siRNA, and shRNA.

12. A reagent kit comprising:

a first oligonucleotide molecule that hybridizes to a target RNA; and
a second oligonucleotide molecule that is hybridized to the first oligonucleotide molecule, and
a reverse transcriptase;
the first oligonucleotide molecule comprising a first region that hybridizes to the target RNA at 3' end, and comprising a second region that is hybridized to the second oligonucleotide molecule, 5' of the first region, wherein the target RNA is a small RNA selected from the group consisting of miRNA, snRNA, snoRNA, piRNA, pri-miRNA, pre-miRNA, siRNA, and shRNA.

13. A combination of a first oligonucleotide molecule that hybridizes to a target RNA a and a reverse transcriptase,
the oligonucleotide molecule comprising a first region at 3' end and a second region 5' of the first region,
the first region hybridizing to the target RNA, and
the second region is hybridized to a second oligonucleotide molecule that is different from the target RNA, wherein the target RNA is a small RNA selected from the group consisting of miRNA, snRNA, snoRNA, piRNA, pri-miRNA, pre-miRNA, siRNA, and shRNA.

## Patentansprüche

1. Verfahren zur Synthese von cDNA durch Mischen einer Ziel-RNA, eines ersten Oligonukleotidmoleküls, eines zweiten Oligonukleotidmoleküls und einer reversen Transkriptase, wobei die Ziel-RNA als Matrize verwendet wird, wobei das erste Oligonukleotidmolekül eine erste Region, die an die Ziel-RNA am 3'-Ende hybridisiert, und eine zweite Region, 5' von der ersten Region, die an das zweite Oligonukleotidmolekül hybridisiert, umfasst, wobei es sich bei der Ziel-RNA um eine kleine RNA ausgewählt aus der Gruppe bestehend aus miRNA, snRNA, snoRNA, piRNA, pri-miRNA, pre-miRNA, siRNA und shRNA handelt.

2. Verfahren nach Anspruch 1, wobei
die erste Region des ersten Oligonukleotidmoleküls an die Ziel-RNA hybridisiert und die zweite Region des ersten Oligonukleotidmoleküls an das zweite Oligonukleotidmolekül hybridisiert und
die reverse Transkriptase das 3'-Ende des ersten Oligonukleotidmoleküls, das mit der Ziel-RNA und dem zweiten Oligonukleotidmolekül hybridisiert ist, verlängert, so dass cDNA synthetisiert wird.

3. Verfahren nach Anspruch 1 oder 2, wobei eine Nukleotidlänge der ersten Region des ersten Oligonukleotidmoleküls 3 oder mehr und 15 oder weniger beträgt.

4. Verfahren nach einem der Ansprüche 1 bis 3, wobei eine Nukleotidlänge der zweiten Region des ersten Oligonukleotidmoleküls 10 oder mehr und 40 oder weniger beträgt.

5. Verfahren nach einem der Ansprüche 1 bis 4, wobei eine Nukleotidlänge des zweiten Oligonukleotidmoleküls 35% oder mehr und 100% oder weniger der Nukleotidlänge der zweiten Region des ersten Oligonukleotidmoleküls beträgt.

6. Verfahren nach einem der Ansprüche 1 bis 5, wobei die Anzahl Basen, die bei Hybridisierung mit der zweiten Region des ersten Oligonukleotidmoleküls eine Fehlpaarungsstelle bilden, im zweiten Oligonukleotidmolekül anteilmäßig innerhalb 50% liegt.

7. Verfahren nach einem der Ansprüche 1 bis 6, wobei das 3'-Ende des zweiten Oligonukleotidmoleküls modifiziert ist, so dass es nicht verlängert wird.

8. Verfahren nach einem der Ansprüche 1 bis 7, ferner umfassend einen Schritt, bei dem die cDNA amplifiziert wird.

9. Verfahren nach Anspruch 8, wobei der cDNA-Amplifikationsschritt Mischen der cDNA, eines Vorwärtsprimers, der an die cDNA hybridisiert, eines Rückwärtsprimers, der an einen Komplementärstrang der cDNA und/oder eines ersten Oligonukleotidmoleküls hybridisiert, und einer Polymerase umfasst.

10. Verfahren nach einem der Ansprüche 1 bis 9, ferner umfassend den Schritt, bei dem wenigstens ein Teil des zweiten Oligonukleotidmoleküls nach der Synthese von cDNA entfernt wird.

11. Verfahren zum Nachweis von Ziel-RNA, umfassend die Schritte:

Synthetisieren von cDNA durch Mischen einer Ziel-RNA, eines ersten Oligonukleotidmoleküls, eines zweiten Oligonukleotidmoleküls und einer reversen Transkriptase, wobei die Ziel-RNA als Matrize verwendet wird; und
Amplifizieren der cDNA und Nachweisen eines Amplifikationsprodukts,
wobei das erste Oligonukleotidmolekül eine erste Region, die an die Ziel-RNA am 3'-Ende hybridisiert, und eine zweite Region, die an das zweite Oligonukleotidmolekül hybridisiert, auf der 5'-Seite der ersten Region umfasst,
wobei es sich bei der Ziel-RNA um eine kleine RNA ausgewählt aus der Gruppe bestehend aus miRNA, snRNA, snoRNA, piRNA, pri-miRNA, pre-miRNA, siRNA und shRNA handelt.

12. Reagentienkit, umfassend:

ein erstes Oligonukleotidmolekül, das an eine Ziel-RNA hybridisiert; und
ein zweites Oligonukleotidmolekül, das an das erste Oligonukleotidmolekül hybridisiert ist, und
eine reverse Transkriptase;
wobei das erste Oligonukleotidmolekül eine erste Region, die an die Ziel-RNA am 3'-Ende hybridisiert, und eine zweite Region, die an das zweite Oligonukleotidmolekül hybridisiert ist, 5' von der ersten Region umfasst, wobei es sich bei der Ziel-RNA um eine kleine RNA ausgewählt aus der Gruppe bestehend aus miRNA, snRNA, snoRNA, piRNA, pri-miRNA, pre-miRNA, siRNA und shRNA handelt.

13. Kombination eines ersten Oligonukleotidmoleküls, das an eine Ziel-RNA hybridisiert, und einer reversen Transkriptase,
wobei das Oligonukleotidmolekül eine erste Region am 3'-Ende und eine zweite Region 5' von der ersten Region umfasst,
wobei die erste Region an die Ziel-RNA hybridisiert und
wobei die zweite Region an ein zweites Oligonukleotidmolekül, das von der Ziel-RNA verschieden ist, hybridisiert ist, wobei es sich bei der Ziel-RNA um eine kleine RNA ausgewählt aus der Gruppe bestehend aus miRNA, snRNA, snoRNA, piRNA, pri-miRNA, pre-miRNA, siRNA und shRNA handelt.

## Revendications

1. Procédé destiné à synthétiser de l'ADNc en mélangeant un ARN cible, une première molécule oligonucléotidique,

une deuxième molécule oligonucléotidique et une transcriptase inverse, en utilisant l'ARN cible comme matrice, la première molécule oligonucléotidique comprenant une première région qui s'hybride avec l'ARN cible à l'extrémité 3', et comprenant une deuxième région, en 5' de la première région, qui s'hybride avec la deuxième molécule oligonucléotidique, dans lequel l'ARN cible est un petit ARN choisi dans le groupe constitué par un ARNmi, un ARNsn, un ARNsno, un ARNpi, un pri-ARNmi, un pré-ARNmi, un ARNsi et un ARNsh.

**2.** Procédé selon la revendication 1, dans lequel
la première région de la première molécule oligonucléotidique s'hybride avec l'ARN cible, et la deuxième région de la première molécule oligonucléotidique s'hybride avec la deuxième molécule oligonucléotidique, et
la transcriptase inverse allonge l'extrémité 3' de la première molécule oligonucléotidique hybridée avec l'ARN cible et la deuxième molécule oligonucléotidique pour synthétiser un ADNc.

**3.** Procédé selon la revendication 1 ou la 2, dans lequel une longueur de nucléotides de la première région de la première molécule oligonucléotidique est de 3 ou plus et de 15 ou moins.

**4.** Procédé selon l'une quelconque des revendications 1 à 3, dans lequel une longueur de nucléotides de la deuxième région de la première molécule oligonucléotidique est de 10 ou plus et de 40 ou moins.

**5.** Procédé selon l'une quelconque des revendications 1 à 4, dans lequel une longueur de nucléotides de la deuxième molécule oligonucléotidique est de 35% ou plus et de 100% ou moins de la longueur de nucléotides de la deuxième région de la première molécule oligonucléotidique.

**6.** Procédé selon l'une quelconque des revendications 1 à 5, dans lequel un rapport du nombre de bases formant un site de mésappariement, quand elles sont hybridées avec la deuxième région de la première molécule oligonucléotidique dans la deuxième molécule oligonucléotidique, se trouve dans les 50%.

**7.** Procédé selon l'une quelconque des revendications 1 à 6, dans lequel l'extrémité 3' de la deuxième molécule oligonucléotidique est modifiée de sorte à ne pas s'allonger.

**8.** Procédé selon l'une quelconque des revendications 1 à 7, comprenant en outre une étape d'amplification de l'ADNc.

**9.** Procédé selon la revendication 8, dans lequel l'étape d'amplification d'ADNc comprend un mélange de l'ADNc, d'une amorce avant qui s'hybride avec l'ADNc, d'une amorce arrière qui s'hybride avec un brin complémentaire de l'ADNc et/ou une première molécule oligonucléotidique, et d'une polymérase.

**10.** Procédé selon l'une quelconque des revendications 1 à 9, comprenant en outre l'étape de retrait d'au moins une partie de la deuxième molécule oligonucléotidique après la synthèse d'ADNc.

**11.** Procédé destiné à détecter un ARN cible, comprenant les étapes :

de synthèse d'ADNc en mélangeant un ARN cible, une première molécule oligonucléotidique, une deuxième molécule oligonucléotidique et une transcriptase inverse, en utilisant l'ARN cible comme matrice ; et
d'amplification de l'ADNc et de détection d'un produit d'amplification,
la première molécule oligonucléotidique comprenant une première région qui s'hybride avec l'ARN cible à l'extrémité 3', et comprenant une deuxième région qui s'hybride avec la deuxième molécule oligonucléotidique sur le côté 5' de la première région, dans lequel l'ARN cible est un petit ARN choisi dans le groupe constitué par un ARNmi, un ARNsn, un ARNsno, un ARNpi, un pri-ARNmi, un pré-ARNmi, un ARNsi et un ARNsh.

**12.** Trousse de réactifs comprenant :

une première molécule oligonucléotidique qui s'hybride avec un ARN cible ; et
une deuxième molécule oligonucléotidique qui est hybridée avec la première molécule oligonucléotidique, et une transcriptase inverse ;
la première molécule oligonucléotidique comprenant une première région qui s'hybride avec l'ARN cible à l'extrémité 3', et comprenant une deuxième région qui est hybridée avec la deuxième molécule oligonucléotidique, en 5' de la première région, dans lequel l'ARN cible est un petit ARN choisi dans le groupe constitué par un ARNmi, un ARNsn, un ARNsno, un ARNpi, un pri-ARNmi, un pré-ARNmi, un ARNsi et un ARNsh.

**13.** Combinaison d'une première molécule oligonucléotidique qui s'hybride avec un ARN cible et une transcriptase inverse,
la molécule oligonucléotidique comprenant une première région à l'extrémité 3' et une deuxième région en 5' de la première région,
la première région s'hybridant avec l'ARN cible et
la deuxième région est hybridée avec une deuxième molécule oligonucléotidique qui est différente de l'ARN cible,
dans laquelle l'ARN cible est un petit ARN choisi dans le groupe constitué par un ARNmi, un ARNsn, un ARNsno, un ARNpi, un pri-ARNmi, un pré-ARNmi, un ARNsi et un ARNsh.

# FIG. 1

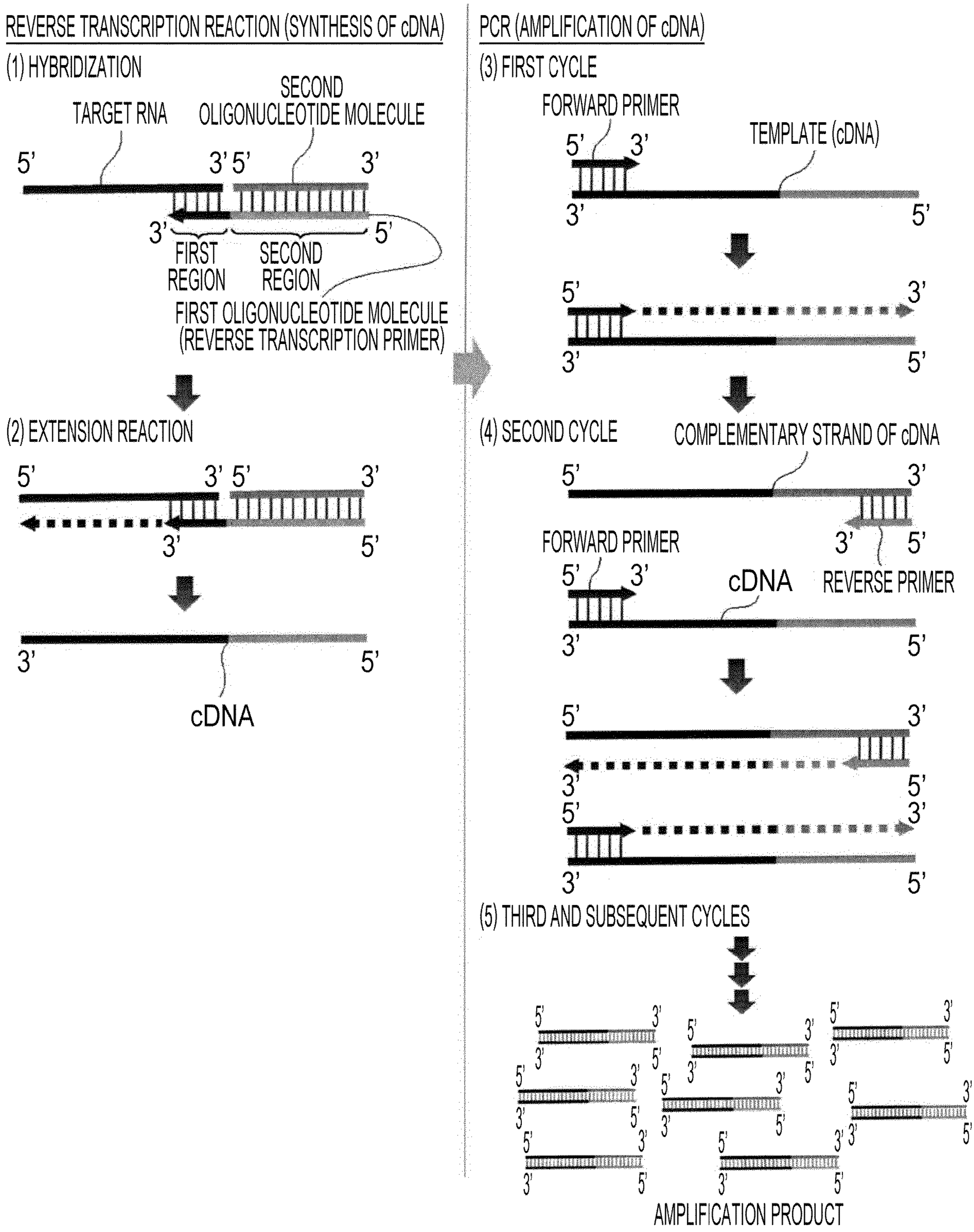
REVERSE TRANSCRIPTION REACTION (SYNTHESIS OF cDNA)
(1) HYBRIDIZATION
TARGET RNA
SECOND OLIGONUCLEOTIDE MOLECULE
5' 3' 5' 3'
3' 5'
FIRST REGION
SECOND REGION
FIRST OLIGONUCLEOTIDE MOLECULE (REVERSE TRANSCRIPTION PRIMER)
(2) EXTENSION REACTION
5' 3' 5' 3'
3' 5'
3' 5'
cDNA
PCR (AMPLIFICATION OF cDNA)
(3) FIRST CYCLE
FORWARD PRIMER
5' 3'
TEMPLATE (cDNA)
3' 5'
(4) SECOND CYCLE
COMPLEMENTARY STRAND OF cDNA
FORWARD PRIMER
cDNA
REVERSE PRIMER
(5) THIRD AND SUBSEQUENT CYCLES
AMPLIFICATION PRODUCT

FIG. 2A

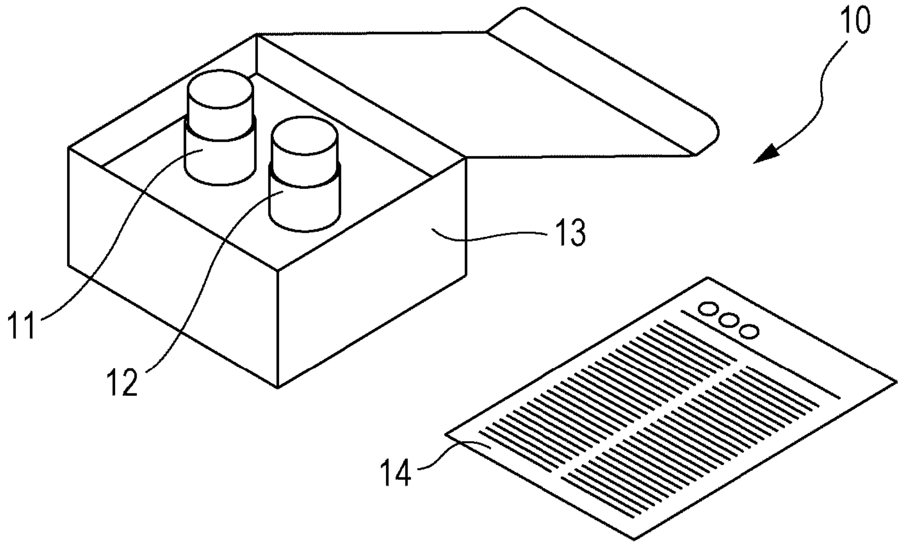


FIG. 2B

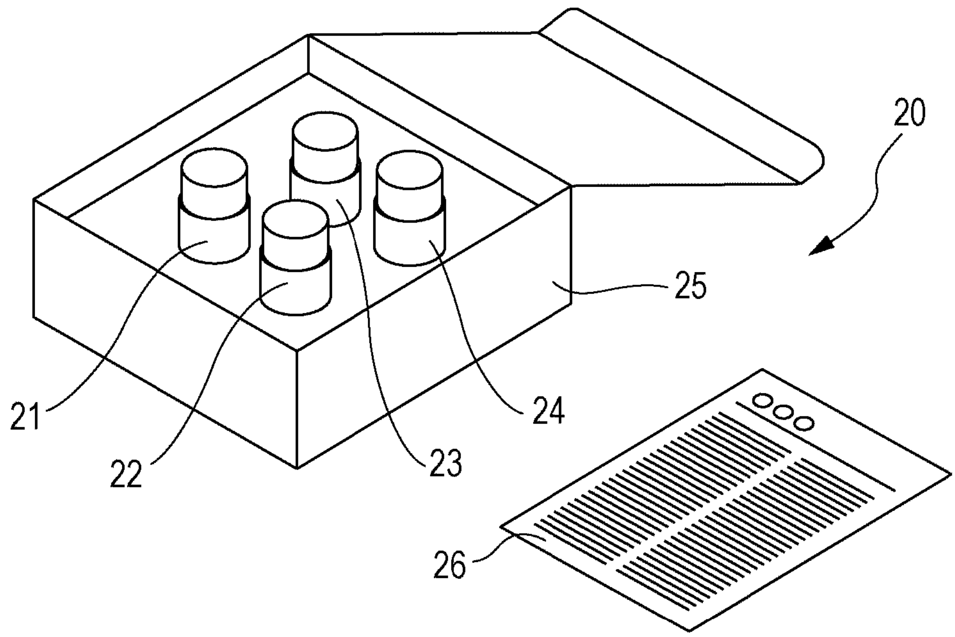

FIG. 2C

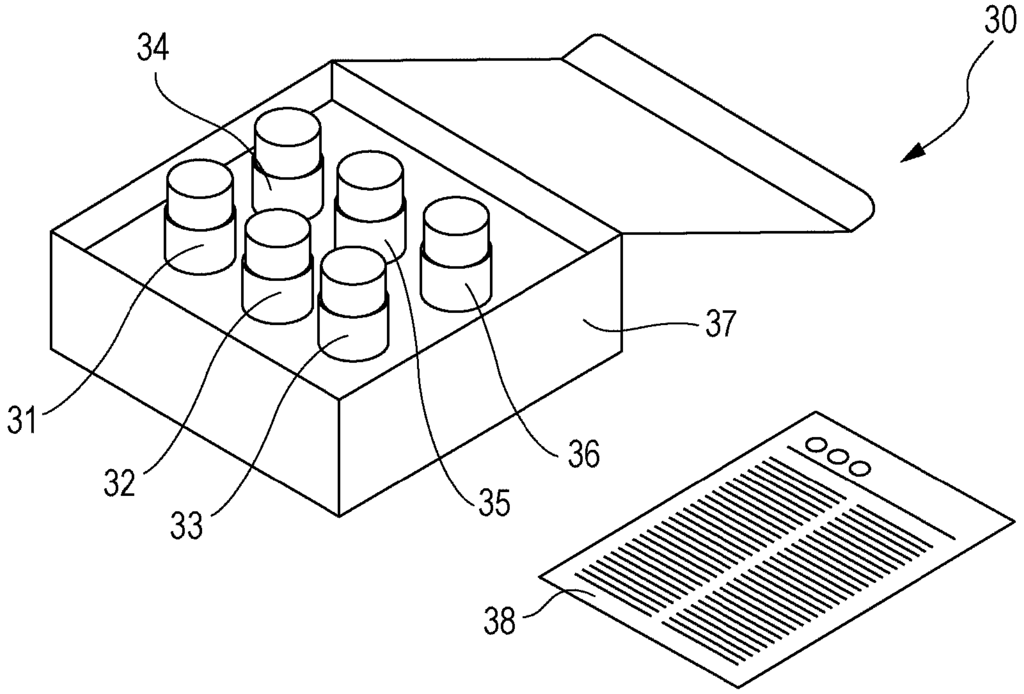


FIG. 2D

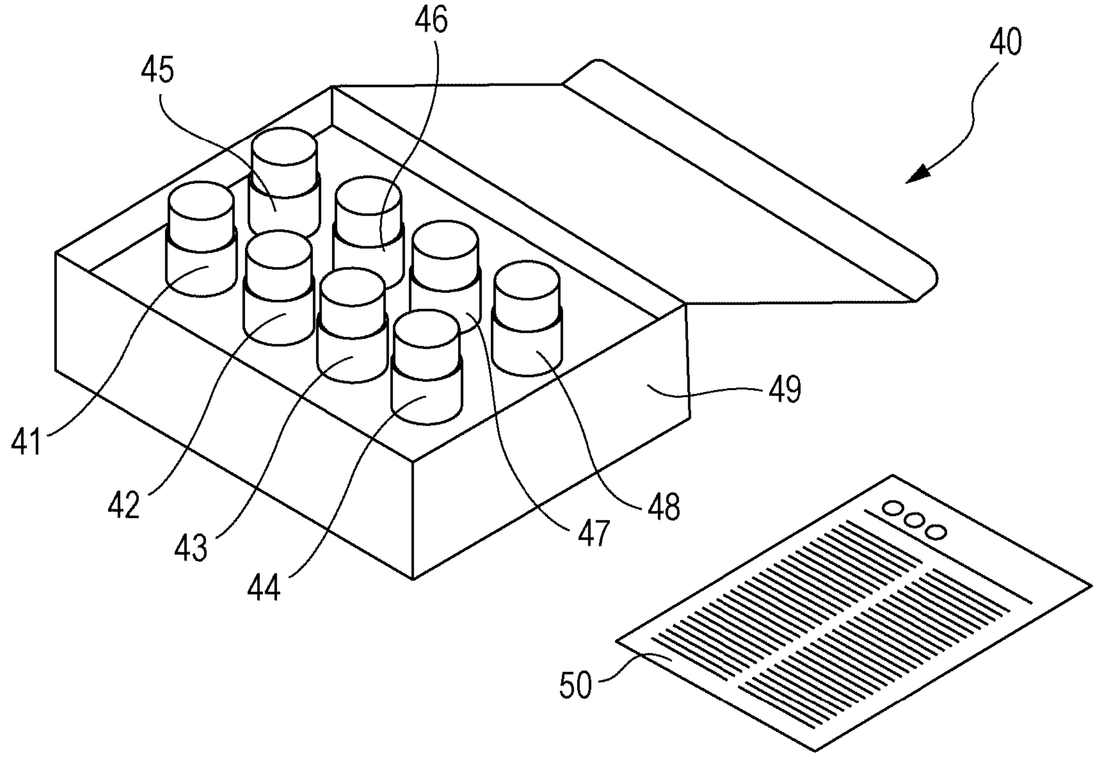

FIG. 3

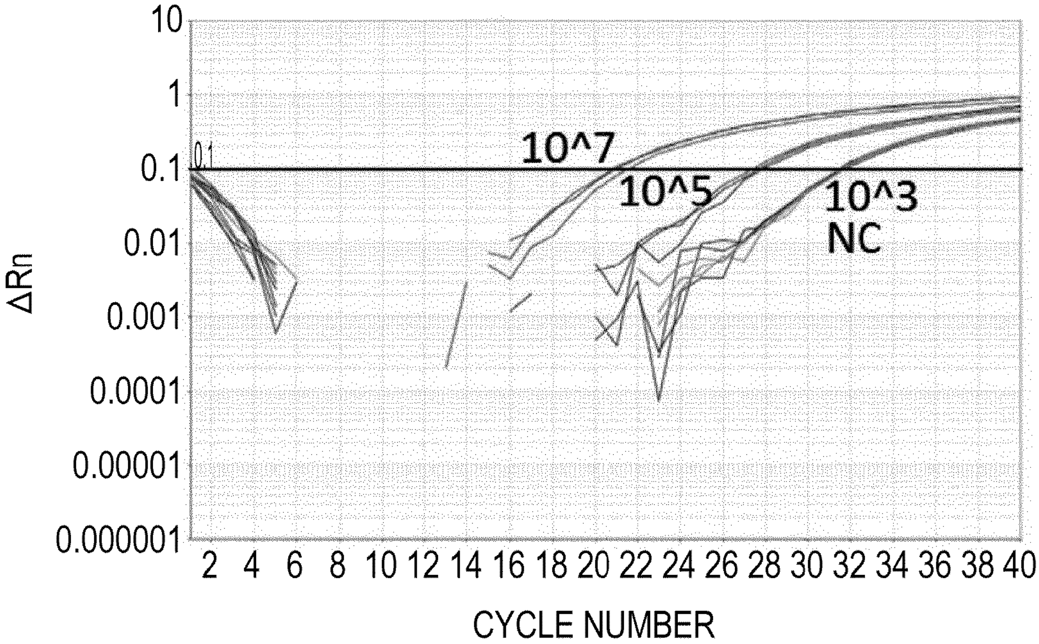
10
1
0.1
0.01
0.001
0.0001
0.00001
0.000001
ΔRn
0.1
10^7
10^5
10^3
NC
2 4 6 8 10 12 14 16 18 20 22 24 26 28 30 32 34 36 38 40
CYCLE NUMBER

FIG. 4

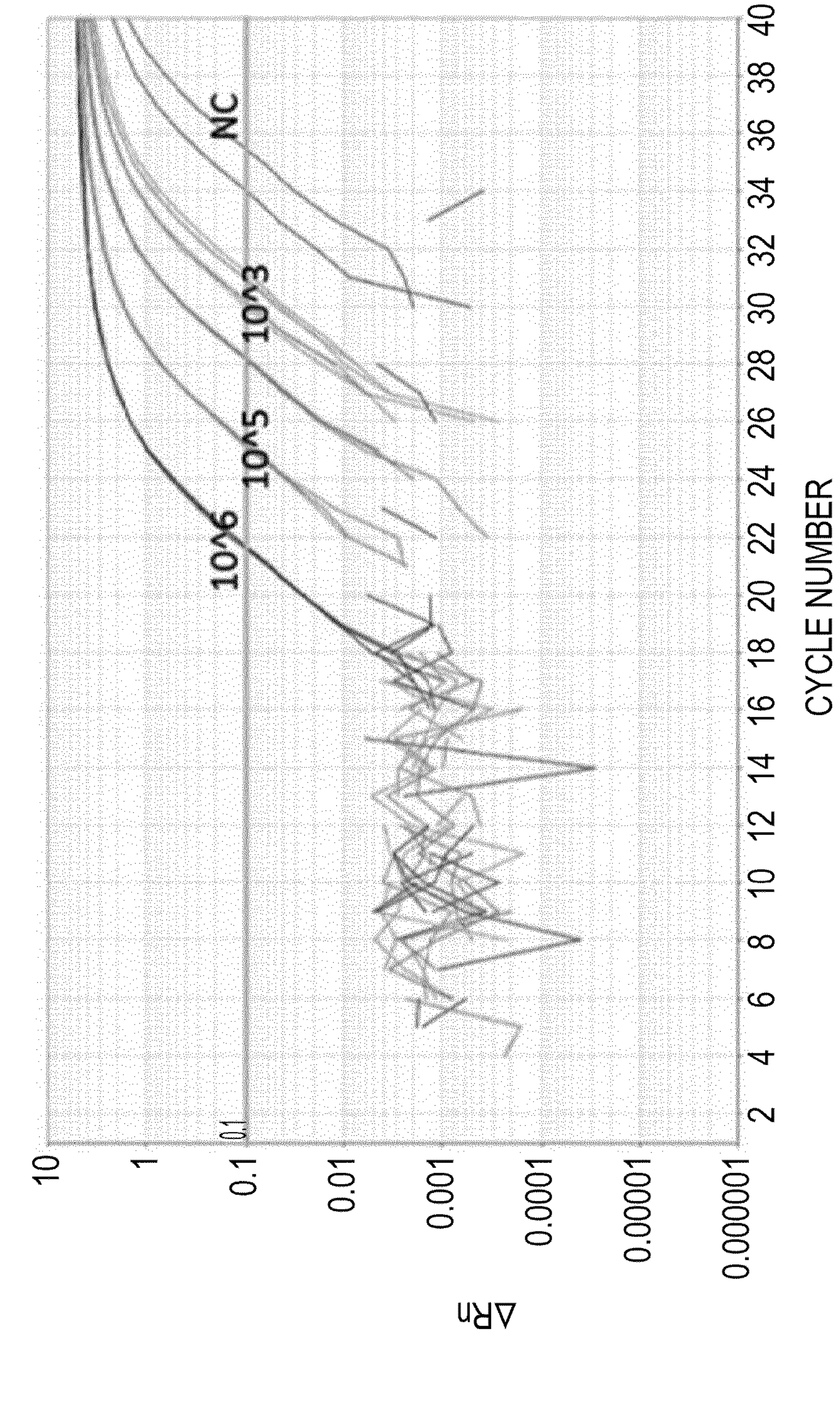
10
1
0.1
0.01
0.001
0.0001
0.00001
0.000001
0.0000001
ΔRn
2 4 6 8 10 12 14 16 18 20 22 24 26 28 30 32 34 36 38 40
CYCLE NUMBER
10^6
10^5
10^3
NC
0.1

*FIG. 5*

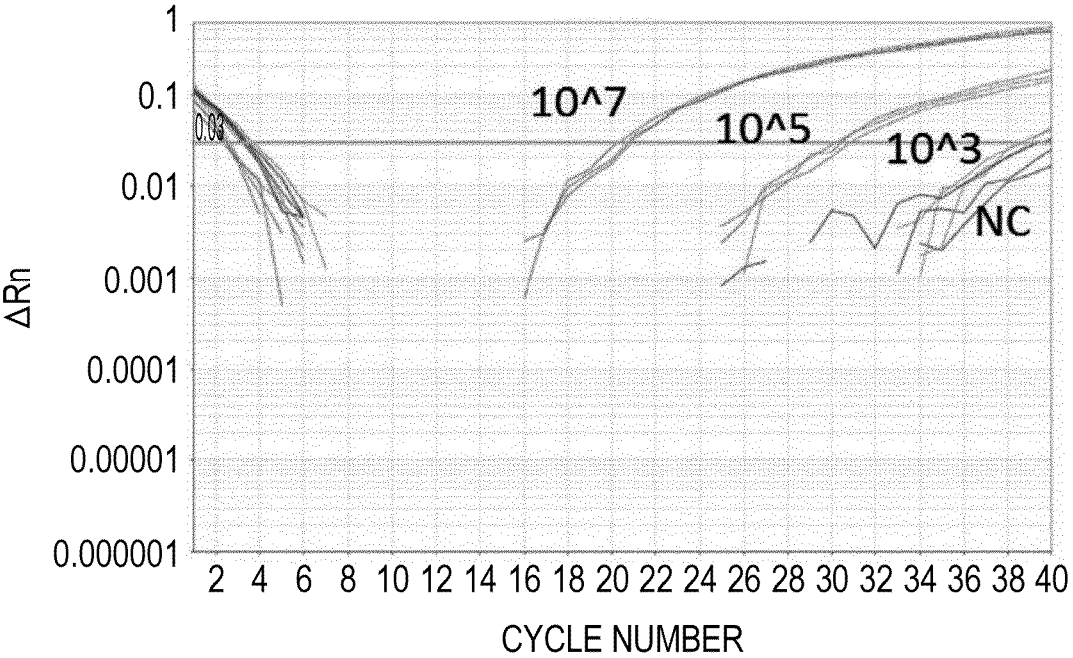
1
0.1
0.01
0.001
0.0001
0.00001
0.000001
ΔRn
0.03
10^7
10^5
10^3
NC
2 4 6 8 10 12 14 16 18 20 22 24 26 28 30 32 34 36 38 40
CYCLE NUMBER

FIG. 6A

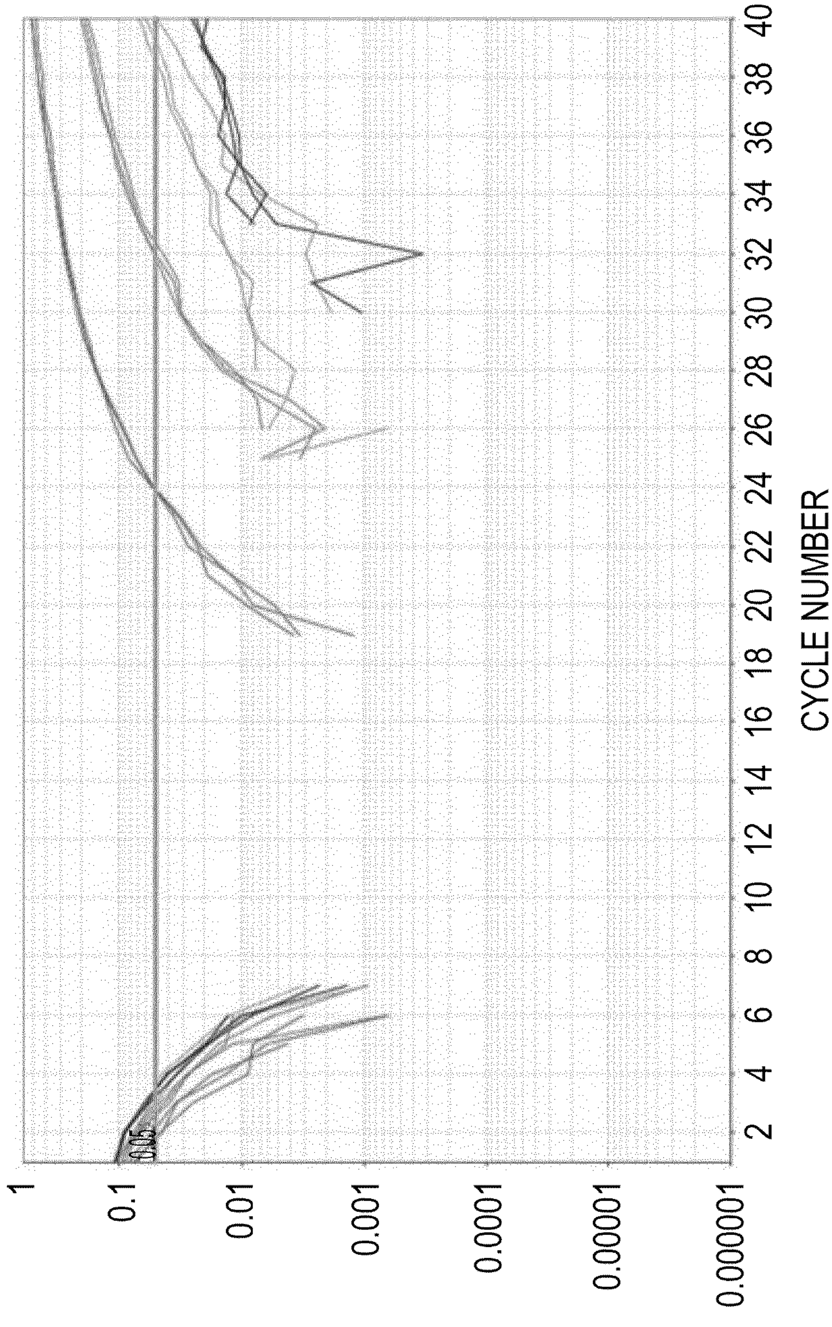

ΔRn
1
0.1
0.01
0.001
0.0001
0.00001
0.000001
0.05
2
4
6
8
10
12
14
16
18
20
22
24
26
28
30
32
34
36
38
40
CYCLE NUMBER

FIG. 6B

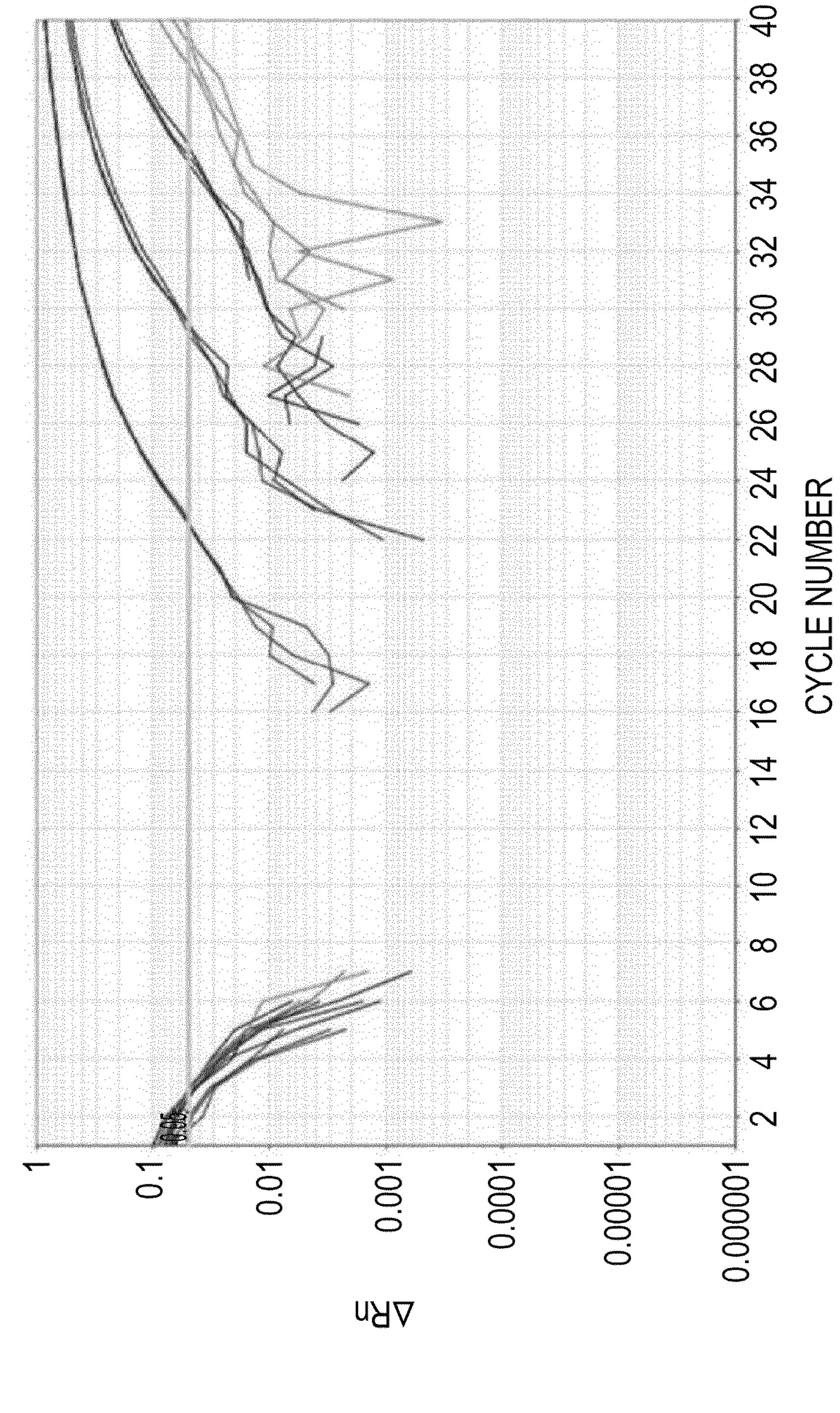
0.05
1
0.1
0.01
0.001
0.0001
0.00001
0.000001
ΔRn
2
4
6
8
10
12
14
16
18
20
22
24
26
28
30
32
34
36
38
40
CYCLE NUMBER

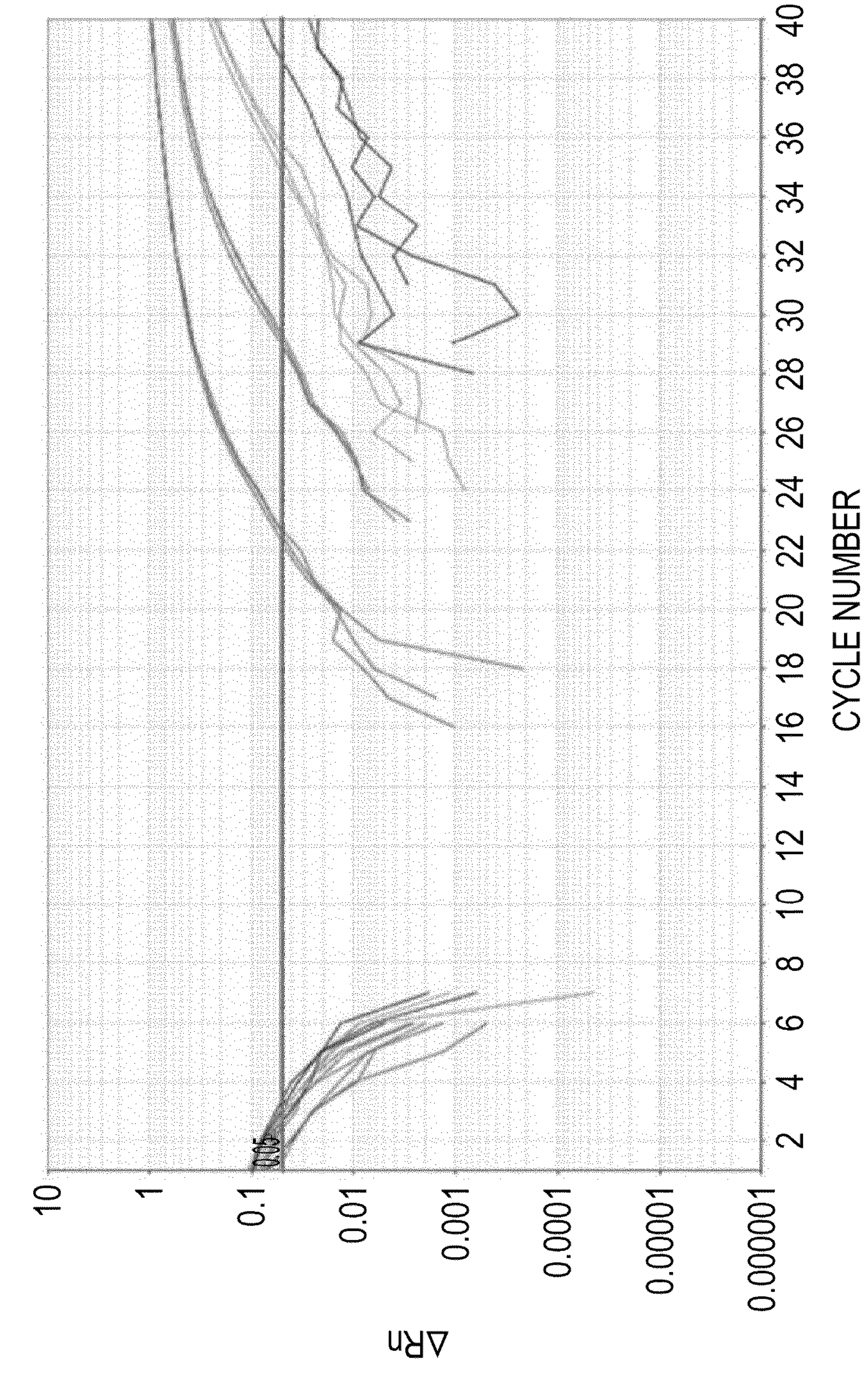

ΔRn
10
1
0.1
0.01
0.001
0.0001
0.00001
0.000001
0.05
2
4
6
8
10
12
14
16
18
20
22
24
26
28
30
32
34
36
38
40
CYCLE NUMBER


FIG. 6C

FIG. 6D

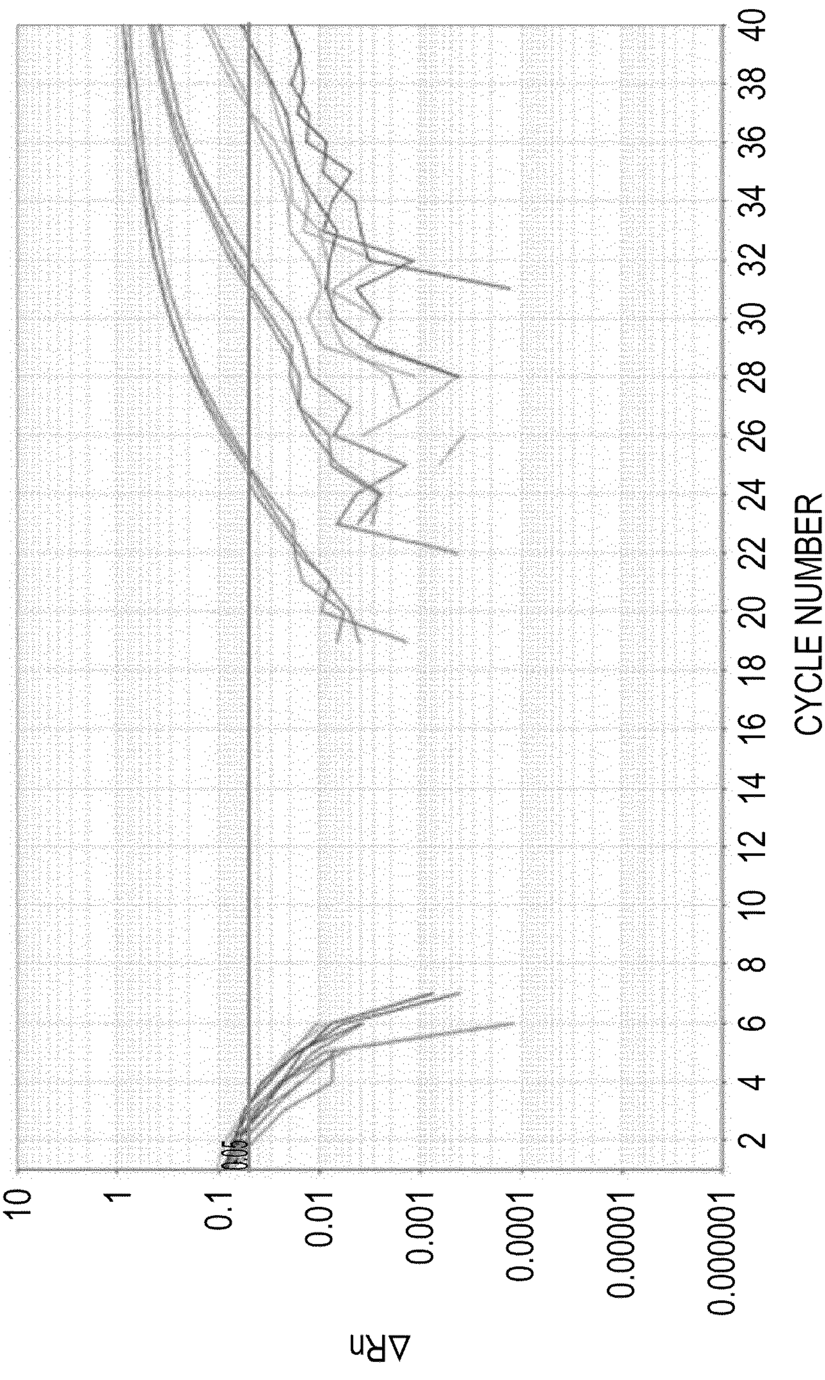
ΔRn
10
1
0.1
0.01
0.001
0.0001
0.00001
0.000001
0.05
2
4
6
8
10
12
14
16
18
20
22
24
26
28
30
32
34
36
38
40
CYCLE NUMBER

FIG. 6E

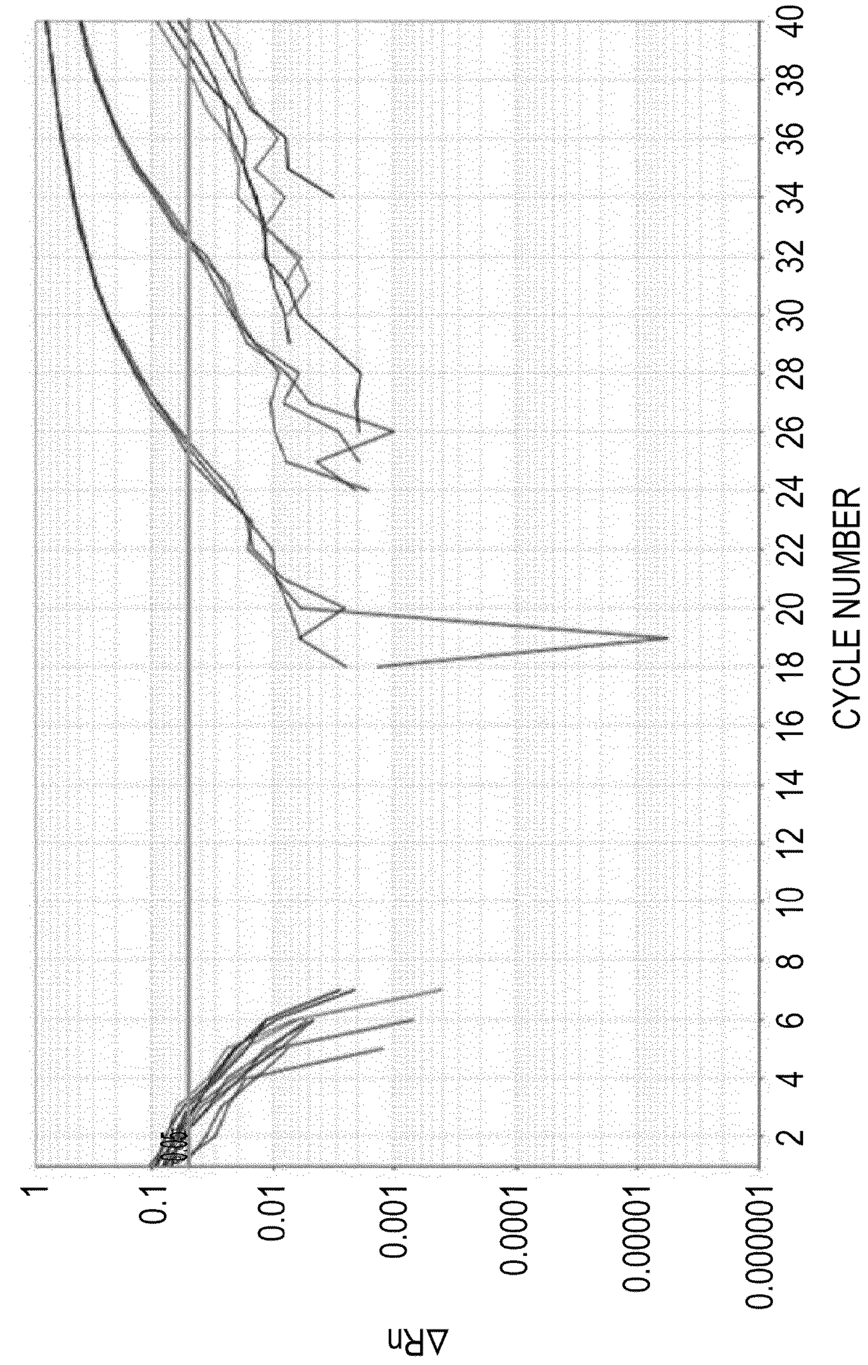
ΔRn
1
0.1
0.01
0.001
0.0001
0.00001
0.000001
CYCLE NUMBER
2 4 6 8 10 12 14 16 18 20 22 24 26 28 30 32 34 36 38 40

FIG. 7A

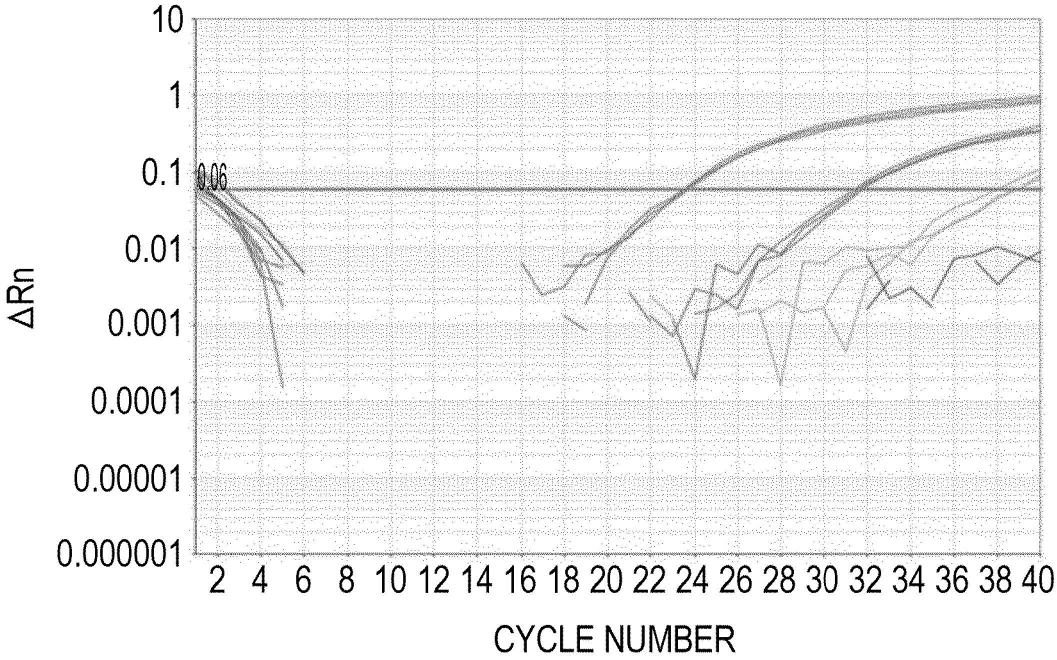
10
1
0.1
0.06
0.01
0.001
0.0001
0.00001
0.000001
ΔRn
2 4 6 8 10 12 14 16 18 20 22 24 26 28 30 32 34 36 38 40
CYCLE NUMBER

FIG. 7B

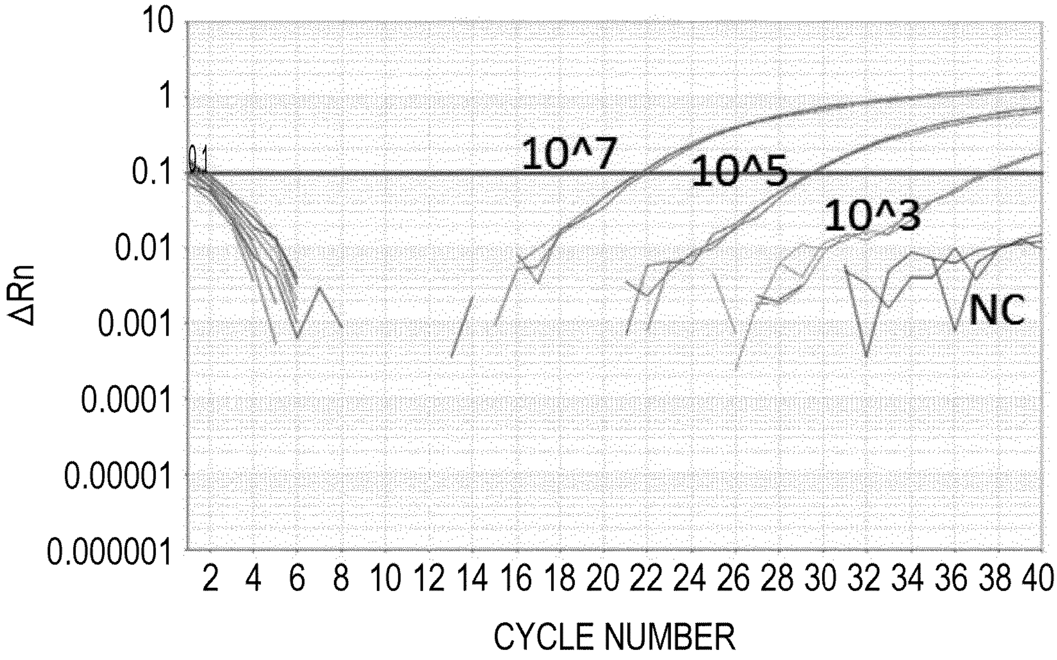
10
1
0.1
0.01
0.001
0.0001
0.00001
0.000001
ΔRn
10^7
10^5
10^3
NC
2 4 6 8 10 12 14 16 18 20 22 24 26 28 30 32 34 36 38 40
CYCLE NUMBER

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1851336 B
- US 7575863 B
- WO 2006003721 A1
- JP 2018103590 A

### Non-patent literature cited in the description

- **SAMBROOK, J. et al.** Molecular Cloning: A Laboratory Manual. Cold Spring Harbor Laboratory Press, 1998